# FASCICULE DE BREVET EUROPEEN

(11) **EP 2 106 401 B1**
(45) Date de publication et mention de la délivrance du brevet: **14.03.2012**
(21) Numéro de dépôt: 08761773.4
(22) Date de dépôt: 17.01.2008
(51) Int. Cl.: C07D 487/04, A61K 31/4188, A61P 29/00

(54) **DERIVES DE PYRROLOPYRIDINE-2-CARBOXAMIDES, LEUR PREPARATION ET LEUR APPLICATION EN THERAPEUTIQUE**
DERIVATE VON PYRROLOPYRIDIN-2-CARBOXAMIDEN, IHRE HERSTELLUNG UND IHRE THERAPEUTISCHE ANWENDUNG
DERIVATIVES OF PYRROLOPYRIDINE-2-CARBOXAMIDS, PREPARATION THEREOF AND THERAPEUTIC APPLICATION THEREOF

(30) Priorité: 19.01.2007 FR 0700356
(43) Date de publication de la demande: 07.10.2009
(73) Titulaire: SANOFI, 75013 Paris (FR)
(72) Inventeur: DUBOIS, Laurent, F-75013 Paris (FR); EVANNO, Yannick, F-75013 Paris (FR); MALANDA, André, F-75013 Paris (FR)
(74) Mandataire: Le Coupanec, Pascale A.M.P.
(86) Numéro de dépôt international: PCT/FR2008/000054
(87) Numéro de publication internationale: WO 2008/107543

(56) Documents cités:
- WO-A-2005/080391
- WO-A1-2007/088277

## Description

L'invention a pour objet des composés dérivés de *N*-hétéroaryl-1-hétéroarylalkyl-1*H-*pyrrolopyridine-2-carboxamides et *N*-hétéroaryl-1-hétéroaryl-1*H*-pyrrolopyridine-2-carboxamides, qui présentent une activité antagoniste *in vitro* et *in vivo* pour les récepteurs de type TRPV1 (ou VR1).

On connaît de WO2005/080391 des dérivés hétérobicycliques tel que le 5-chloro-7-(3-méthyl-2-pyridyl)(4-triflluorométhylphényl)[1,2,4]triazolo[4,3-a]pyridin-3-amine, utiles comme antagonistes des récepteurs de type VR1.

Un premier objet de l'invention concerne les composés répondant à la formule générale (I) ci-après.

Un autre objet de l'invention concerne des procédés de préparation des composés de formule générale (I).

Un autre objet de l'invention concerne l'utilisation des composés de formule générale (I) notamment dans des médicaments ou dans des compositions pharmaceutiques.

Les composés de l'invention répondent à la formule générale (I) 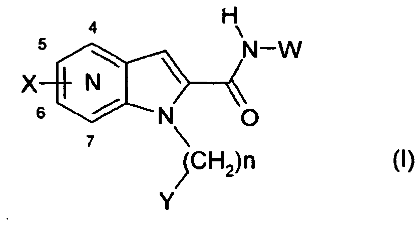 dans laquelle
n est égal à 0,1, 2 ou 3 ;
le noyau pyrrolopyridine est un groupe pyrrolo[3,2-*b*]pyridine, un groupe pyrrolo[3,2-c]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et / ou 7 par un ou plusieurs substituants X, identiques ou différents l'un de l'autre, choisis parmi un atome d'halogène ou un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cyocloalkyle-C₁-C₃-alkylène-O-, C₁-C₈-fluoroalcoxyle, cyano, C(O)NR₁R₂, nitro, NR₁R₂, C₁-C₆-thioalkyle, -S(O)-C₁-C₆-alkyle, -S(O)₂-C₁-C₆-alkyle, SO₂NR₁R₂, NR₃COR₄, NR₃SO₂R₅, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène-O-, C₁-C₆-fluoroalkyle, C₁-C₆-alcoxyle, C₃-C₇-cycloalcoxyle, C₃-C₇-cycloalkyle-C₁-C₃-alkylène-O-, C₁-C₆-fluoroalcoxyle, nitro ou cyano ;
Y représente un hétéroaryle éventuellement substitué par un ou plusieurs groupes choisis parmi un atome d'halogène ou un groupe C₁-C₆-alkyle, C₃-C₇-cycloalkyle, C₃-C₇-cycloalkyle-C₁-C₃-alkylène, C₁-C₆-fluoroalkyle, hydroxyle, C₁-C₆-alcoxyle, C₃-C₇-cycloalcoxyle, C₃-C₇-cycloalkyle-C₁-C₆-alkylène-O-, C₁-C₆-fluoroalcoxyle, cyano, C(O)NR₁R₂, nitro, NR₁R₂, C₁-C₆-thioalkyle, C₁-C₆-thiofluoroalkyle, thiol, -S(O)-C₁-C₆-alkyle, -S(O)-C₃-C₇-cycloalkyle, -S(O)-C₁-C₆-alkylène-C₃-C₇-cycloalkyle, -S(O)₂-C₁-C₆-alkyle, -S(O)₂-C₃-C₇-cycloalkyle, -S(O)₂-C₁-C₆-alkylène-C₃-C₇-cycloalkyle SO₂NR₁R₂, NR₃COR₄, NR₃SO₂R₅, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
R₁ et R₂, représentent, indépendamment l'un de l'autre, un atome d'hydrogène ou un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
ou R₁ et R₂ forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
R₃ et R₄ représentent, indépendamment l'un de l'autre, un atome d'hydrogène ou un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène; C₁-C₆-fluoroalkyle, C₁-C₆-alcoxyle, C₃-C₇-cycloalcoxyle, C₃-C₇-cycloalkyle-C₁-C₈-alkylène-O-, C₁-C₆-fluoroalcoxyle, nitro ou cyano ;
R₅ représente un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃. alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
W représente un groupe bicyclique fusionné de formule: 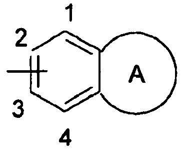 lié à l'atome d'azote par les positions 1, 2, 3 ou 4 ;
A représente un hétérocycle de 5 à 7 chaînons comprenant de un à trois hétéroatomes choisis parmi O, S ou N ;
le ou les atomes de carbone de A étant éventuellement substitués par un ou plusieurs groupes choisis parmi un atome d'hydrogène ou un groupe C₁-C₆-alkyle, C₃-C₇-cycloalkyle, C₃-C₇-cycloalkyle-C₁-C₃-alkylène, C₁-C₆-fluoroalkyle, aryle, aryl-C₁-C₅-alkylène, hétéroaryle ou hétéroaryl-C₁-C₅-alkylène, oxo ou thio; l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
le ou les atomes d'azote de A étant éventuellement substitués par R₆ lorsque l'azote est adjacent à un atome de carbone substitué par un groupe oxo, ou par R₇ dans les autres cas ;
R₆ représente un atome d'hydrogène ou un groupe C₁-C₆-alkyle, C₃-C₇-cydoalkyle, C₃-C₇-cycloalkyle-C₁-C₃-alkylène, C₁-C₆-fluoroalkyle, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;

R₇ représente un atome d'hydrogène ou un groupe C₁-C₆-alkyle, C₃-C₇-cycloalkyle, C₃-C₇-cycloalkyle-C₁-C₃-alkylène, C₁-C₆-fluoroalkyle, aryle-C₁-C₆-alkylène, C₁-C₆-alkyle-C(O)-, C₃-C₇-cycloalkyle-C₁-C₃-alkylène-(CO)-, C₁-C₆-fluoroalkyle-C(O)-, C₃-C₇-cycloalkyle-C(O)-, aryle-C(O)-, aryle-C₁-C₆-alkylène-C(O)-, C₁-C₆-alkyle-S(O)₂-, C₁-C₆-fluoroalkyle-S(O)₂-, C₃-C₇-cycloalkyle-S(O)₂-, C₃-C₇-cycloalkyle-C₁-C₃-alkylène-S(O)₂-, aryle-S(O)₂- ou aryle-C₁-C₆-alkylène-S(O)₂. ou aryle.

Dans les composés de formule générale (I) :
- le ou les atomes de soufre de l'hétérocycle A peuvent être sous forme oxydée (S(O) ou S(O)₂) ;
- le ou les atomes d'azote de l'hétérocycle A peuvent être sous forme oxydée (N-oxyde) ;
- l'atome d'azote en position 4, 5, 6 ou 7 de la pyrrolopyridine peut être sous forme oxydée (N-oxyde).

Dans le cadre de l'invention, on peut citer à titre d'exemple de groupe W les groupes indolinyle, isoindolinyle, indolyle, isoindolyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[e][1,4]diazépinyle, tétrahydrobemo[b][1,4]oxazépinyle ou tétrahydrobenzo[b][1,4]thiazépinyle ;
ces groupes pouvant être éventuellement substitués comme défini dans la formule générale (I).

Dans le cadre de la présente invention on entend par :
- Cₜ-C_{z} où t et z peuvent prendre les valeurs de 1 à 7, une chaîne carbonée pouvant avoir de t à z atomes de carbone, par exemple C₁-C₃ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe C₁₋₃-alkylène représente une chaîne carbonée divalente de 1 à 3 atomes de carbone, linéaire ou ramifiée, par exemple un méthylène, éthylène, 1-méthyléthylène, propylène,
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un cycloalcoxyle : un radical -O-cycloalkyle où le groupe cycloalkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un thiofluoroalkyle : un groupe thioalkyle dont un ou plusieurs atomes d'hydrogène ont été substitués par un atome de fluor ;
- un aryle : un groupe aromatique cyclique comprenant entre 6 et 10 atomes de carbones. A titre d'exemples de groupes aryles, on peut citer les groupes phénate ou naphtyle ;
- un hétéroaryle : un groupe cyclique aromatique de 5 à 10 chaînons contenant de 1 à 4 hétéroatomes choisis parmi O, S ou N. A titre d'exemple, on peut citer les groupes imidazolyle, thiazolyle, oxazolyle, furanyle, thiophényle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle, quinoxalinyle cinnolinyle, quinazolinyle, phtalazinyle, naphtyridinyle ;
- un hétérocycle : un groupe cyclique saturé, partiellement insaturé ou aromatique de 5 à 7 chaînons, comprenant de un à trois hétéroatomes choisis parmi O, S ou N;
- un atome d'halogène : un fluor, un chlore, un brome ou un iode ;
- « oxo » signifie « =O » ;
- « thio » signifie « =S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Parmi les composés de formule générale (I) objets de l'invention, un premier sous-groupe de composés est constitué par les composés pour lesquels, si n est égal à 0, alors le ou les substituents X, identiques ou différents l'un de l'autre, sont choisis parmi un atome d'halogène ou un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₃-alkylène-O-, C₁-C₆-fluoroalcoxyle, cyano, C(O)NR₁R₂, nitro, NR₁R₂, C₁-C₆-thioalkyle,-S(O)-C₁-C₆-alkyle, -S(O)₂-C₁-C₆-alkyle, SO₂NR₁R₂, NR₃COR₄, NR₃SO₂R₅.

Parmi les composés de formule générale (I) objets de l'invention, un second sous-groupe de composés est constitué par les composés pour lesquels n est égal à 0 ou 1.

Parmi les composés de formule générale (I) objets de l'invention, un troisième sous-groupe de composés est constitué par les composés pour lesquels
le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et / ou 7 par un ou plusieurs substituants X, identiques ou différents l'un de l'autre, choisis parmi un atome d'halogène ou un groupe C₁-C₆-alkyle, C₁-C₆-fluoroalkyle.

Parmi les composés de formule générale (I) objets de l'invention, un quatrième sous-groupe de composés est constitué par les composés pour lesquels
le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 5 par un substituant X tel que défini dans la formule générale (I).

Parmi les composés de formule générale (I) objets de l'invention, un cinquième sous-groupe de composés est constitué par les composés pour lesquels
le noyau pyrrolopyridine est un groupe pyrrolo[2,3-*b*]pyridine éventuellement substitué en position carbonée 4, 5, 6 et / ou 7 par un ou plusieurs substituants X, identiques ou différents l'un de l'autre, choisis parmi un atome d'halogène, plus particulièrement de fluor, ou un groupe C₁-C₆-fluoroalkyle, plus particulièrement un trifluorométhyle.

Parmi les composés du cinquième sous-groupe, un sixième sous-groupe de composés est constitué par les composés pour lesquels
le noyau pyrrolopyridine est un groupe pyrrolo[2,3-*b*]pyridine éventuellement substitué en position carbonée 5 par un substituant X choisi parmi un atome d'halogène, plus particulièrement de fluor, ou un groupe C₁-C₆-fluoroalkyle, plus particulièrement un trifluorométhyle.

Parmi les composés de formule générale (I) objets de l'invention, un septième sous-groupe de composés est constitué par les composés pour lesquels
Y représente un groupe choisi parmi les groupes imidazolyle, thiazolyle, oxazolyle, furanyle, thiophényle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle, quinoxalinyle, cinnolinyle, quinazolinyle, phtalazinyle, naphtyridinyle ; ce groupe étant éventuellement substitué comme défini dans la formule générale (I).

Parmi les composés du septième sous-groupe, un huitième sous-groupe de composés est constitué par les composés pour lesquels Y représente un pyridinyle.

Parmi les composés de formule générale (I) objets de l'invention, un neuvième sous-groupe de composés est constitué par les composés pour lesquels
W est choisi parmi les groupes indolinyle, isoindolinyle, indolyle, isoindolyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépin-yle, tétrahydrobenzo[e][1,4]-diazépinyle, tétrahydrobenzo[*b*][1,4]oxazépinyle ou tétrahydrobenzo[*b*][1,4]thiazépinyle ; ces groupes pouvant être éventuellement substitués comme défini dans la formule générale (I).

Parmi les composés de formule générale (I) objets de l'invention, un dixième sous-groupe de composés est constitué par les composés pour lesquels
W représente un groupe benzimidazolyle ou benzothiazolyle,
le ou les atomes de carbones de W étant éventuellement substitués par un ou plusieurs groupes C₁-C₆-alkyle, plus particulièrement méthyle ;
le ou les atomes d'azote de W étant éventuellement substitués par un groupe C₁-C₅-alkyle, plus particulièrement méthyle.

Les composés pour lesquels à la fois n, X, Y et W sont tels que définis dans les sous-groupes de composés de formule générale (I) ci-dessus, forment un onzième sous-groupe.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.

Selon le schéma 1, les composés de formule générale (IV) peuvent être obtenus par réaction d'un composé de formule générale (II) dans laquelle X est tel que défini dans la formule générale (I) ci-dessus et B représente un groupe hydroxyle ou C₁-C₆-alcoxyle, avec un composé de formule générale (III), dans laquelle Y et n sont tels que définis dans la formule générale (I) ci-dessus et R' représente un groupe partant ou un groupe hydroxyle.

Lorsque le composé de formule générale (III), est défini tel que n est égal à 1, 2 ou 3 et R' représente un groupe partant tel qu'un atome de brome ou d'iode, la réaction peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1),1).

Lorsque le composé de formule générale (III), est défini tel que n est égal à 0 et R' représente un groupe partant tel qu'un atome de brome, de chlore ou d'iode, la réaction peut être réalisée par application et adaptation des méthodes décrites par S. L. Buchwald et al. (J. Am. Chem. Soc., 2001, 123, 7727 et 2002, 124, 11684), de préférence sous atmosphère inerte en milieu basique, par exemple en présence de triphosphate de potassium, en présence d'un sel de cuivre tel que l'iodure de cuivre, éventuellement en présence d'un additif tel que la *N,N'*-diméthylcyclohexane-1,2-diamine, le tout dans un solvant organique tel que le toluène.

Lorsque le composé de formule générale (III) est défini tel que n est égal à 1, 2 ou 3 et R' représente un groupe hydroxyle, les composés de formule générale (IV), peuvent être obtenus par réaction du composé de formule générale (II) avec un composé de formule générale (III) en présence d'une phosphine telle que la triphénylphosphine et d'un réactif tel que l'azodicarboxylate de diéthyle en solution dans un solvant tel que le dichlorométhane ou le tétrahydrofurane (O. Mitsonobu, Synthesis, 1981, 1-28).

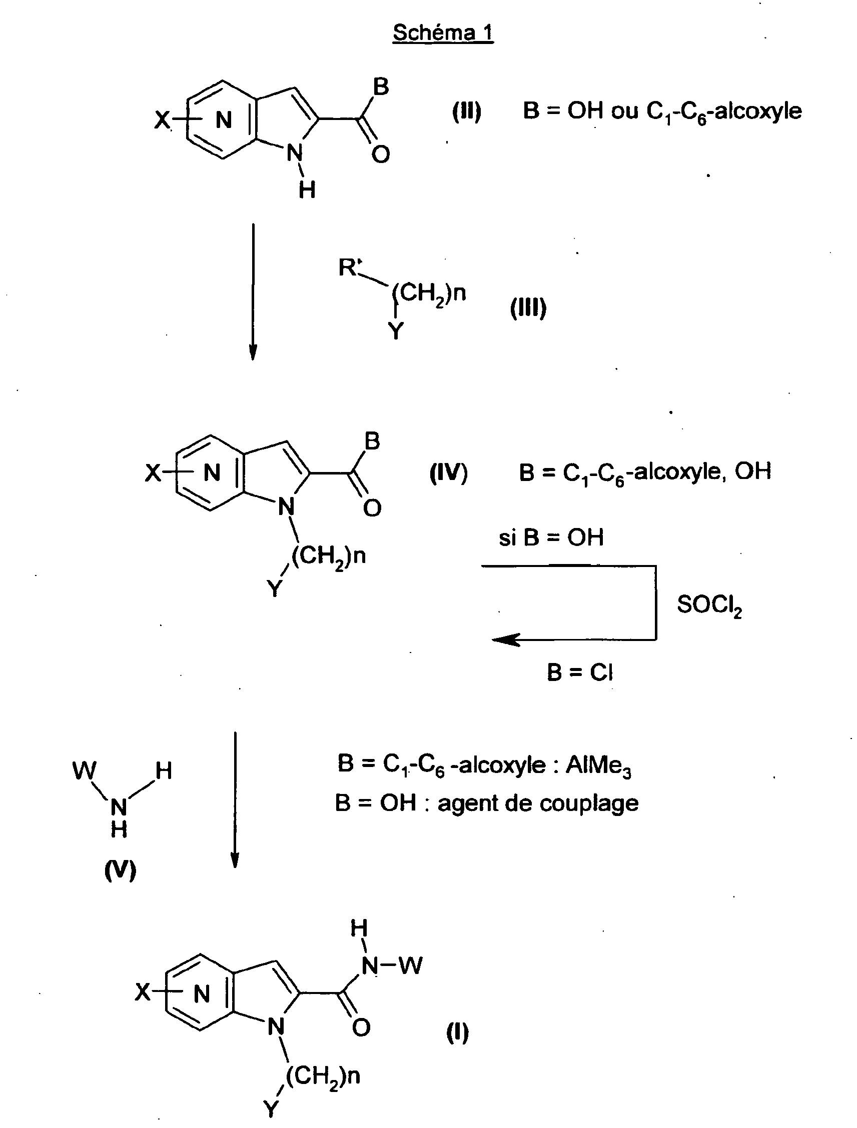

Dans le cadre de l'invention, les composés de formule générale (IV) dans laquelle B représente un groupe C₁-C₆-alcoxyle peuvent être transformés en composés de formule générale (IV) dans laquelle B représente un groupe hydroxyle selon des méthodes connues de l'homme du métier, par exemple en présence d'une base telle que l'hydroxyde de sodium dans un solvant tel que le méthanol ou l'éthanol à une température comprise entre 20°C et la température d'ébullition du milieu réactionnel. Dans le cadre de l'invention, les composés de formule générale (IV) dans laquelle B représente une groupe hydroxyle peuvent être transformés en composés de formule générale (IV) dans laquelle B représente un groupe C₁-C₆-alcoxyle selon des méthodes connues de l'homme du métier, par exemple en présence d'un acide tel que l'acide sulfurique dans un solvant tel que le méthanol ou l'éthanol.

Dans le cas des composés de formule générale (IV), dans laquelle B représente un groupe C₁-C₆-alcoxyle, le composé de formule générale (I) peut être obtenu par réaction d'un composé de formule générale (IV), tel qu'obtenu ci-dessus, avec un amidure du composé de formule générale (V), dans laquelle W est tel que défini dans la formule générale (I) ci-dessus, au reflux d'un solvant tel que le toluène. L'amidure d'aluminium du composé de formule générale (V) est préparé par action préalable du triméthylaluminium sur les amines de formule générale (V).

Dans le cadre de l'invention, les composés de formule générale (IV), dans laquelle B représente un groupe hydroxyle, peuvent réagir avec les composés de formule générale (V) pour conduire aux composés de formule générale (I) en présence d'un agent de couplage tel qu'un dialkylcarbodiimide, l'hexafluorophosphate de [(benzotriazol-1-yl)oxy][tris(pyrrolidino)]phosphonium, le diéthylcyanophosphonate par exemple, en présence d'une base comme la triéthylamine, dans un solvant inerte tel que le diméthylformamide, ou selon les méthodes de couplage de la chimie peptidique (M. Bodanszky et al., principes of peptide synthesis, Springer-Verlag, New York, NY, 1984, 9-58).

Dans le cas des composés de formule générale (IV), dans laquelle B représente un groupe hydroxyle, la fonction acide carboxylique peut préalablement être transformée en halogénure d'acide tel qu'un chlorure d'acide par action du chlorure de thionyle, de préférence sous atmosphère inerte (par exemple sous azote ou sous argon) au sein d'un solvant tel que l'acetonitrile, le dichlorométhane, le dichloroéthane ou le toluène, à une température comprise entre 20°C et la température d'ébullition du milieu réactionnel comme décrit dans la littérature (par exemple dans WO2006059163). Le composé de formule générale (I) est alors obtenu par réaction du composé de formule générale (IV), dans laquelle B représente un atome de chlore, avec le composé de formule générale (V), en présence d'une base telle que la triéthylamine ou le carbonate de sodium.

Dans le schéma 1, les composés de formule (II), (III) et (V) et les autres réactifs, quand leur mode de préparation n'est pas décrit, sont disponibles dans le commerce, décrits dans la littérature ou préparés par analogie à de nombreux procédés décrits dans la littérature (Merck WO2006089309 ; P. Roy et al Synthesis 2005, 16, 2751-2757; N. Lahance et al Synthesis 2005, 15, 2571-2577 ; C. Barberis et al Tetrahedron Lett 2005, 46(51), 8877-8880 ; T. Lomberget Synlett 2005, 13, 2080-2082 ; 1909 ; M. Nazare et al Angew Chem Int Ed 2004, 43(34), 4526-4528 ; P.M. Fresneda et al Tetrahedron Lett 2000, 41(24), 4777-4780; M.H. Fisher et al J Heterocyclic Chem 1969, 6, 775; B. Frydman et al J Am Chem Soc 1965, 87, 3530 ; L.N. Yakhontov Tetrahedron Lett 1969, 1909 ; B. frydman J Org Chem 1968, 33(10), 3762 ; G.P. Fagan et al J Med Chem 1988 31(5), 944 ; OSI Pharmaceuticals WO2004104001 ; WO03049702; US0149367 ; WO03068749, par exemple).

Les composés de formules générales (II), (IV) ou (I), dans lesquelles X représente un groupe alkyle peuvent être obtenus par une réaction de couplage, catalysée par un métal tel que le palladium ou le fer, réalisée sur les composés de formules générales (II), (IV) ou (I) correspondants, dans lesquelles X représente un atome d'halogène, par exemple un chlore, en présence par exemple d'un halogénure d'alkylmagnésium ou d'un halogénure d'alkylzinc selon les méthodes décrites dans la littérature (A. Furstner et al J Am Chem Soc 2002, 124(46), 13856 ; G. Quéguiner et al J Org Chem 1998, 63(9), 2892 par exemple) ou connues de l'homme de métier.

Les composés de formules générales (II), (IV) et (I), dans lesquelles X représente un groupe cyano ou un aryle, peuvent être obtenus par une réaction de couplage, catalysée par un métal tel que le palladium, réalisée sur les composés de formules générales (II), (IV) ou (1) correspondants, dans lesquelles X représente par exemple un atome de brome, en présence de cyanure de triméthylsilyle ou d'acide arylboronique, ou par toutes autres méthodes qui sont décrites dans la littérature ou qui sont connues de l'homme du métier.

Les composés de formules générales (I), (II) et (IV), dans lesquelles X représente un groupe NR₁R₂, NR₃COR₄ ou NR₃SO₂R₅, peuvent être obtenus à partir des composés de formules générales (I), (II) et (IV) correspondants, dans lesquelles X représente, par exemple, un atome de brome, par réaction de couplage respectivement avec une amine, un amide ou une sulfonamide en présence d'une base, d'une phosphine et d'un catalyseur à base de palladium, selon des méthodes qui sont décrites dans la littérature ou qui sont connues de l'homme du métier.

Les composés de formules générales (II), (IV) et (I), dans lesquelles X représente un groupe C(O)NR₁R₂, peuvent être obtenus à partir des composés de formules générales (II), (IV) ou (I) correspondants, dans lesquelles X représente un groupe cyano, selon des méthodes qui sont décrites dans la littérature ou qui sont connues de l'homme du métier. Les composés de formules générales (II), (IV) et (I), dans lesquelles X représente un groupe -S(O)-alkyle ou -S(O)₂-alkyle, peuvent être obtenus par oxydation des composés de formules générales (II), (IV) ou (I) correspondants, dans lesquelles X représente un groupe C₁-C₆-thioalkyle, selon des méthodes qui sont décrites dans la littérature ou qui sont connues de l'homme du métier.

Les composés de formules générales (II), (IV) et (I), dans lesquelles X représentent un groupe NR₁R₂, NR₃COR₄ ou NR₃SO₂R₅, peuvent être obtenus à partir des composés de formules générales (II), (IV) ou (I) correspondants, dans lesquelles X représente un groupe nitro, par exemple par réduction, puis acylation ou sulfonylation, selon des méthodes qui sont décrites dans la littérature ou qui sont connues de l'homme du métier. Les composés de formules générales (II), (IV) et (I), dans lesquelles X représente un groupe SO₂NR₁R₂ peuvent être obtenus par une méthode analogue à celle décrite dans *Pharmazie* **1990**, *45*, 346, ou selon des méthodes qui sont décrites dans la littérature ou qui sont connues de l'homme du métier.

Les composés de formule générale (I), dans laquelle R₇ représente un atome d'hydrogène peuvent être obtenus à partir des composés de formule générale (I) dans laquelle, par exemple, R₇ représente un groupe phénylméthyle, par hydrogénation en présence d'un catalyseur à base de palladium ou par toutes méthodes qui sont décrites dans la littérature ou qui sont connues de l'homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 1. Les microanalyses élémentaires, les analyses LC-MS (chromatographie liquide couplée à la spectrométrie de masse) les spectres I.R. et/ou les spectres R.M.N. confirment les structures des composés obtenus.

### Exemple 1 (composé n°1)

### N-(2-méthyl-benzothiazol-5-yl)-5-fluoro-1-[(4-pyridyl)méthyl]-1H-pyrrolo[2,3-b]pyridine-2-carboxamide

### 1.1 2-amino-3-iodo-5-fluoropyridine

Dans un bicol de 500 mL, muni d'un agitateur magnétique sont introduits, 5 g (44,6 mmoles) de 2-amino-5-fluoropyridine, 13,9 g (44,6 mmoles) de sulfate d'argent et 400 mL d'éthanol. On ajoute ensuite, par petite portion, 11,31 g (44,6 mmoles) d'iode en poudre. L'agitation est poursuivie à température ambiante pendant 24 heures. La suspension jaune résultante est filtrée, le précipité est rincé à l'éthanol et le filtrat est concentré sous pression réduite. Le résidu ainsi obtenu est repris dans un mélange d'acétate d'éthyle (200 mL) et d'une solution saturée en carbonate de sodium (200 mL). Après séparation, la phase organique est lavée successivement avec une solution aqueuse de thiosulfate de sodium à 25% et avec une solution aqueuse saturée en chlorure de sodium puis séchée sur sulfate de sodium et concentrée sous pression réduite. Le solide résultant est purifié par chromatographie sur colonne de silice en éluant avec un mélange de n-heptane et d'acétate d'éthyle. On obtient 2,67 g du produit attendu.
R.M.N. ¹H (DMSO D₆), δ (ppm) : 7,9 (m, 2H) ; 5,9 (s, 2H).

### 1.2 acide 5-fluoro-1H-pyrrolo[2,3-b]pyridine-2-carboxylique

Dans un tube scellé de 100 mL, muni d'un agitateur magnétique et maintenu sous bullage d'argon, sont introduits, 2 g (7,98 mmoles) de 2-amino-3-iodo-5-fluoropyridine, obtenu selon le protocole décrit à l'étape 1.1, 2,1 g (23,95 mmoles) d'acide pyruvique, 2,68 g (23,95 mmoles) de 1,4-diazabicyclo[2.2.2]octane (DABCO) et 50 mL de diméthylformamide anhydre. Après quelques minutes, on ajoute 0,18 g (0,8 mmole) d'acétate de palladium. Le mélange réactionnel est maintenu sous agitation et sous bullage d'argon pendant 20 minutes puis rapidement scellé et porté à 100°C pendant 3h. La solution refroidie est concentrée sous pression réduite. Le résidu est ensuite repris par de l'acétate d'éthyle (200 mL), on lave la phase organique trois fois avec 100 mL d'eau puis elle est extraite par trois fois 50 mL d'une solution aqueuse de soude 2N. Les phases aqueuses basiques sont rassemblées, refroidies à 0°C puis acidifiées par ajout d'acide chlorhydrique (pH 3). On extrait ensuite par trois fois 100 mL d'acétate d'éthyle. Les phases organiques rassemblées sont séchées sur du sulfate de sodium puis concentrées sous pression réduite. On obtient 0,87 g du produit attendu sous forme d'une poudre jaune.
R.M.N. ¹H (DMSO D₆), δ (ppm) : 13,2 (s, 1H) ; 12,4 (s, 1H) ; 8,4 (dd, 1H) ; 7,95 (dd, 1H) ; 7,1 (d, 1H).

### 1.3 5-fluoro-1H-pyrrolo[2,3-b]pyridine-2-carboxylate d'éthyle

Dans un ballon de 100 mL, muni d'un agitateur magnétique, sont introduits 0,9 g (5 mmoles) d'acide 5-fluoro-1*H-*pyrrolo[2,3-*b*]pyridine-2-carboxylique obtenu selon le protocole décrit à l'étape 1.2 et 10 mL d'éthanol. On ajoute 0,5 mL d'acide sulfurique concentré au mélange réactionnel que l'on porte ensuite à reflux pendant 24 heures. La solution refroidie est concentrée sous pression réduite. Le résidu est repris par du dichlorométhane (200 mL) que l'on lave successivement avec une solution aqueuse d'hydrogénocarbonate de sodium (2 x 100 mL), avec de l'eau (50 mL) puis avec 50 mL d'une solution aqueuse saturée en chlorure de sodium. On sèche la phase organique sur sulfate de sodium puis on la concentre sous pression réduite. On isole 0,87 g du produit attendu.
R.M.N. ¹H (DMSO D₆), δ (ppm): 12,6 (s, 1H) ; 8,4 (dd, 1H) ; 8,0 (dd, 1H) ; 7,1 (d, 1H) ; 4,3 (q, 2H) ; 1,3 (t, 3H).

### 1.4 5-fluoro-1-[(4-pyridyl)méthyl]-1H-pyrrolo[2,3-b]pyridine-2-carboxylate d'éthyle

A une solution de 0,25 g (1,2 mmole) du 5-fluoro-1*H*-pyrrolo[2,3-*b*]pyridine-2-carboxylate d'éthyle obtenu selon le protocole décrit à l'étape 1.3 dans 10 mL de tétrahydrofurane sec maintenue sous atmosphère inerte, sont ajoutés successivement sous agitation, 0,19 g (1,8 mmole) de 4-pyridylméthanol puis 0,48 g (1,8 mmole) de triphénylphosphine. On ajoute ensuite, goutte à goutte à 0°C, 0,32 g (1,8 mmole) d'azodicarboxylate de diéthyle. Le mélange réactionnel est alors agité pendant 72h à température ambiante puis concentré sous pression réduite. On purifie l'huile résultante par chromatographie sur colonne de gel de silice en éluant avec un mélange d'heptane et d'acétate d'éthyle. On isole 0,26 g du produit attendu.
R.M.N. ¹H (DMSO D₆), δ (ppm) : 8,5 (m, 1H) ; 8,4 (d, 2H) ; 8,1 (dd, 1H) ; 7,4 (s, 1H) ; 6,95 (d, 2H); 5,9 (s, 2H) ; 4,3 (q, 2H), 1,2 (t, 3H).

### 1.5 N-(2-méthyl-benzothiazol-5-yl)-5-fluoro-1-[(4-pyridyl)méthyl]-1H-pyrrolo[2,3-b]pyridine-2-carboxamide (composé n°1)

Dans un tricol de 50 mL, refroidi à 0°C et muni d'une agitation magnétique, sont introduits sous balayage d'azote, 0,19 g (1,15 mmole) de 5-amino-2-méthylbenzothiazole et 5 mL de toluène sec. A cette solution, on ajoute ensuite lentement 0,72 mL (1,44 mmole) d'une solution 2M de triméthylaluminium dans le toluène. Le mélange réactionnel obtenu est maintenu sous atmosphère d'azote et agité en laissant la température progressivement atteindre 70°C. On ajoute alors, goutte à goutte en 5 minutes, une solution de 0,28 g (0,93 mmole) de 5-fluoro-1-[(4-pyridyl)méthyl]-1*H*-pyrrolo[2,3-*b*]pyridine-2-carboxylate d'éthyle obtenu selon le protocole décrit à l'étape 1.4, dans 5 mL de toluène sec. Le mélange réactionnel est alors porté à reflux pendant 3 heures. Après retour à température ambiante, la suspension obtenue est refroidie à 0°C puis hydrolysée par ajout, goutte à goutte, de 20 mL d'eau froide suivie de l'addition de 5 mL d'acide chlorhydrique 1 N. Après 30 minutes d'agitation, le pH du mélange réactionnel est ajusté à 8 par ajout d'une solution saturée en hydrogénocarbonate de sodium. On extrait ensuite à l'acétate d'éthyle. Les phases organiques rassemblées sont lavées successivement avec une solution saturée aqueuse d' hydrogénocarbonate de sodium, à l'eau puis avec une solution saturée de chlorure de sodium. Après séchage sur sulfate de sodium, filtration et concentration sous pression réduite du filtrat, on obtient une huile qui cristallise peu à peu au repos. Le solide ainsi formé est trituré dans un minimum de dichlorométhane, filtré puis agité dans de l'ether diisopropylique à chaud pour donner après filtration et séchage 0,127 g du produit attendu sous la forme d'un solide beige.
Point de fusion : 217 - 219 °C
R.M.N. ¹H (DMSO D₆), δ (ppm) : 10,7 (s, 1H) ; 8,45 (m, 3H) ; 8,3 (d, 1H) ; 8,2 (dd, 1H) ; 7,9 (d, 1H) ; 7,7 (dd, 1H) ; 7,5 (s, 1H) ; 7,0 (d, 2H) ; 5,9 (s, 2H) ; 2,8 (s, 3H).

### Exemple 2 (composé n°2)

### N-(1,2-diméthyl-1H-benzimidazol-5-yl)-5-fluoro-1-[(4-pyridyl)méthyl]-1H-pyrrolo[2,3-b]pyridine-2-carboxamide

Dans un tricol de 50 mL, refroidi à 0°C et muni d'une agitation magnétique, sont introduits sous balayage d'azote, 0,18 g (1,15 mmole) de 5-amino-1,2-diméthyl-1*H-*benzimidazole et 5 mL de toluène sec. A cette solution, on ajoute ensuite lentement 0,72 mL (1,44 mmole) d'une solution 2M de triméthylaluminium dans le toluène. Le mélange réactionnel obtenu est maintenu sous atmosphère d'azote et agité en laissant la température progressivement atteindre 70 °C. On ajoute alors, goutte à goutte en 5 minutes, une solution de 0,3 g (0,96 mmole) de 5-fluoro-1-[(4-pyridyl)méthyl]-1*H*-pyrrolo[2,3-*b*]pyridine-2-carboxylate d'éthyle obtenu selon le protocole décrit à l'étape 1.4, dans 5 mL de toluène sec. Le mélange réactionnel est porté à reflux pendant 18 heures. Après retour à température ambiante, la suspension obtenue est refroidie à 0°C puis hydrolysée par ajout, goutte à goutte, de 20 mL d'eau froide suivie de l'addition de 5 mL d'acide chlorhydrique 1 N. Après 30 minutes d'agitation, le pH du mélange réactionnel est ajusté à 8 par ajout d'une solution saturée d'hydrogénocarbonate de sodium. On extrait ensuite à l'acétate d'éthyle. Les phases organiques rassemblées sont lavées successivement avec une solution saturée aqueuse d'hydrogénocarbonate de sodium, à l'eau puis avec une solution saturée de NaCl. On sèche ensuite sur sulfate de sodium et on filtre sur fritté. On observe la formation progressive d'un précipité blanc. On le recueille, on le rince avec de l'acétate d'éthyle et on le sèche sous vide. On obtient 0,157 g du produit attendu sous la forme d'un solide blanc.
Point de fusion : 263 - 265 °C
R.M.N. ¹H (DMSO D₆), δ (ppm): 10,4 (s, 1H) 8,4 (m, 3H) ; 8,15 (dd, 1H) ; 7,9 (m, 1H) ; 7,45 (m, 3H) ; 7,0 (d, 2H) ; 5,9 (s, 2H) ; 3,7 (s, 3H) ; 2,5 (s, 3H).

### Exemple 3 (Composé n°3)

### N-(1,2-diméthyl-1H benzimidazol-5-yl)-1-[(4-pyridyl)méthyl]-1H-pyrrolo[2,3-b]pyridine-2-carboxamide

### 3.1 2-(t-butyloxycàrbonylamino)-3-méthylpyridine

Dans un tricol de 100 mL équipé d'un agitateur magnétique, on introduit 31 g (142,03 mmoles) de dicarbonate de ditertbutyle et 35 mL d'hexane que l'on porte au reflux. On ajoute alors, goutte à goutte sur une période de 2 heures, une solution de 10 g (88,77 mmoles) de 2-amino-3-méthylpyridine dans 10 mL d'acétate d'éthyle. Le reflux est maintenu 1h après la fin de l'addition. Après retour à température ambiante, on ajoute 20 mL d'hexane et le précipité blanc formé après agitation du mélange réactionnel est recueilli par filtration, rincé à l'hexane et séché sous pression réduite. On obtient 15,5 g de cristaux blancs.
R.M.N. ¹H (CDCl₃), δ (ppm) : 8,3 (dd, 1H) ; 7,5 (dd, 1H) ; 7,4 (s, 1H) ; 7,1 (ddd, 1H) ; 2,3 (s, 3H) ; 1,5 (s, 9H).

### 3.2 1H-pyrrolo[2,3-b]pyridine-2-carboxylate d'éthyle

Dans un tricol de 250 mL, équipé d'un agitateur magnétique et maintenu sous atmosphère d'azote, on introduit 5 g (24,01 mmoles) de 2-(*t*-butyloxycarbonylamino)-3-méthylpyridine obtenu selon le protocole décrit à l'étape 3.1 et 50 mL de tétrahydrofurane sec. On ajoute, goutte à goutte en maintenant la température inférieure à 5°C, 30 mL (48,02 mmoles) d'une solution de n-butyllithium 1,6 M dans le THF. Après 1h d'agitation à 0°C, le dérivé lithié ainsi obtenu est ajouté à une solution de 7,08 g (48,02 mmoles) d'oxalate de diéthyle dans 50 mL de tétrahydrofurane sec maintenue à une température de -3°C. On laisse ensuite revenir le milieu réactionnel à température ambiante, puis on le verse sur une solution de 25 mL d'acide chlorhydrique 6N refroidie à 0°C en maintenant la température inférieure à 10°C. Le mélange obtenu est ensuite agité à 50°C pendant 2 heures puis à température ambiante pendant la nuit. Le milieu réactionnel est ajusté à pH 3 par de la soude et est extrait à l'éther diéthylique. On sèche la phase organique sur du sulfate de sodium, on la filtre et on la concentre sous pression réduite. On obtient 1,8 g de produit utilisé tel quel dans les étapes suivantes.
R.M.N. ¹H (CDCl₃), δ (ppm) : 8,8 (dd, 1H) ; 8,15 (dd, 1H); 7,2 (m, 2H) ; 4,5 (q, 2H) ; 1,5 (t, 3H).

### 3.3 1-[(4-pyridyl)méthyl]-1H-pyrrolo[2,3-b]pyridine-2-carboxylate d'éthyle

A une solution de 0,6 g (3,15 mmoles) de 1*H*-pyrrolo[2,3-*b*]pyridine-2-carboxylate d'éthyle obtenu selon le protocole décrit à l'étape 3.2, dans 40 mL de tétrahydrofurane sec, maintenue sous atmosphère inerte, sont ajoutés successivement sous agitation, 0,51 g (4,73 mmoles) de 4-pyridylméthanol puis 1,24 g (4,73 mmoles) de triphénylphosphine. On ajoute ensuite, goutte à goutte à 0°C, 0,82 g (4,73 mmoles) d'azodicarboxylate de diéthyle. Le mélange réactionnel est agité pendant 24 h à température ambiante puis concentré sous pression réduite. On ajoute un mélange d'eau et d'acétate d'éthyle (20 mL, v/v) et on ajuste le pH du mélange réactionnel à 5-6 par ajout d'acide acétique. La phase organique séparée est ensuite lavée deux fois avec 10 mL d'eau et une fois avec 10 mL d'une solution saturée de chlorure de sodium puis séchée sur du sulfate de magnesium. Après concentration sous pression réduite, on purifie l'huile résultante par chromatographie sur colonne de gel de silice. On isole 0,66 g de produit attendu.
R.M.N. ¹H (COCl₃), δ (ppm) : 8,5 (m, 3H) ; 8,1 (dd, 1H) ; 7,35 (s, 1H) ; 7,2 (m, 1H) ; 7,0 (d, 2H) ; 6,0 (s, 2H) ; 4,3 (q, 2H) ; 1,3 (t, 3H).

### 3.4 N-(1,2-Diméthyl-1H-benzimidazol-5-yl)-1-[(4-pyridyl)méthyl]-1H pyrrolo[2,3-b]pyridine-2-carboxamide (composé n°3)

Dans un Keller de 50 mL muni d'une agitation magnétique, est introduit sous balayage d'argon, 0,189 g (1,17 mmole) de 5-amino-1,2-diméthyl-1*H*-benzimidazole et 10,6 mL de toluène. On ajoute ensuite goutte à goutte 0,85 mL (1,71 mmole) d'une solution 2M de triméthylaluminium dans le toluène à température ambiante. Le mélange réactionnel est porté à 50°C pendant 30 mn puis on ajoute 0,3 g (1,07 mmole) du 1-[(4-pyridyl)méthyl]-1*H*-pyrrolo[2,3-*b*]pyridine-2-carboxylate d'éthyle obtenu selon le protocole décrit à l'étape 3.3. Le mélange réactionnel est alors porté à reflux pendant 5 heures. Après retour à température ambiante, on verse le mélange dans la soude normale et on extrait trois fois avec 10 mL d'acétate d'éthyle. Les phases organiques rassemblées sont lavées successivement par 10 mL d'eau et par 10 mL d'une solution aqueuse saturée de chlorure de sodium, séchées sur sulfate de sodium puis concentrées sous pression réduite. Le solide résultant est purifié par chromatographie sur colonne de silice en éluant avec un mélange de dichlorométhane et de méthanol. Le solide isolé est ensuite recristallisé dans l'isopropanol. On obtient 0,2 g du produit attendu.
Point de fusion : 236 - 246 °C
R.M.N. ¹H (DMSO D₆), δ (ppm) : 8,4 (m, 3H) ; 8,2 (dd, 1H) ; 7,85 (d, 1H) ; 7,4 (m, 3H) 7,2 (dd, 1H) ; 7,0 (d, 2H) ; 5,9 (s, 2H) ; 3,7 (s, 3H) ; 2,5 (s, 3H).

### Exemple 4 (composé n°4)

### N-(2-méthyl-benzothiazol-5-yl)-1-[(4-pyridyl)méthyl]-1H-pyrrolo[2,3-b]pyridine-2-carboxamide

Dans un Keller de 50 mL muni d'une agitation magnétique, est introduit sous balayage d'argon, 0,224 g (1,37 mmole) de 5-amino-2-méthylbenzothiazole et 12,44 ml de toluène. On ajoute ensuite goutte à goutte 1 mL (1,99 mmole) d'une solution 2M de triméthylaluminium dans le toluène à température ambiante. Le mélange réactionnel est porté à 50°C pendant 30 mn puis on ajoute, goutte à goutte en 5 mn, une solution de 0,35 g (1,24 mmole) du 1-[(4-pyridyl)méthyl]-1*H*-pyrrolo[2,3-*b*]pyridine-2-carboxylate d'éthyle obtenu selon le protocole décrit à l'étape 3.3 dans 12,44 mL de toluène sec. Le mélange réactionnel est alors porté à reflux pendant 14 heures. Après retour à température ambiante, on rajoute au milieu réactionnel 1 mL (1,99 mmole) d'une solution 2M de triméthylaluminium dans le toluène et on porte à nouveau le mélange réactionnel à reflux pendant 7 heures. Après retour à température ambiante, on verse le mélange dans la soude normale et on extrait trois fois avec 10 mL d'acétate d'éthyle. Les phases organiques rassemblées sont lavées successivement par 10 mL d'eau et par 10 mL d'une solution aqueuse saturée en chlorure de sodium, séchées sur sulfate de sodium puis concentrées sous pression réduite. Le solide résultant est purifié par chromatographie sur colonne de silice en éluant avec un mélange de dichlorométhane et d'acétate d'éthyle. Le solide isolé est ensuite recristallisé dans l'isopropanol. On obtient 0,276 g du produit attendu.
Point de fusion : 211 - 236 °C
R.M.N. ¹H (DMSO D₆), δ (ppm) : 8,5 (m, 3H) ; 8,1 (m, 3H) ; 7,8 (d, 1H) ; 7,7 (dd, 1H) ; 7,25 (3, 3H) ; 7,1 (s, 1H) ; 6,1 (s, 2H) ; 2,8 (s, 3H).

### Exemple 5 (composé n°5)

### N-(2-méthyl-benzothiazol-5-yl)-5-trifluorométhyl-1-[(4-pyridyl)méthyl]-1H-pyrrolo[2,3-b]pyridine-2-carboxamide

### 5.1 2-amino-3-iodo-5-trifluorométhyl-pyridine

Dans un bicol de 500 mL, muni d'un agitateur magnétique, sont introduits 2 g (12,34 mmoles) de 2-amino-5-trifluorométhylpyridine, 3,85 g (12,34 mmoles) de sulfate d'argent et 80 mL d'éthanol. On ajoute ensuite, par petites portions, au milieu réactionnel agité à température ambiante, 3,13 g (12,34 mmoles) d'iode en poudre. Le mélange réactionnel est alors agité à température ambiante pendant 48 heures. La suspension jaune résultante est filtrée, le précipité est rincé à l'éthanol et le filtrat évaporé sous pression réduite. Le résidu est repris avec 100 mL de dichlorométhane. On lave cette solution successivement avec 20 mL d'une solution aqueuse de soude à 5%, 20 mL d'eau et 20 mL d'une solution aqueuse saturée en chlorure de sodium puis on la sèche sur sulfate de sodium et on la concentre sous pression réduite. Le solide résultant est purifié par chromatographie sur colonne de silice en éluant avec un mélange de n-heptane et d'acétate d'éthyle. On obtient 1,71 g de produit sous la forme d'une poudre rose.
R.M.N. ¹H (DMSO D₆), δ (ppm) : 8,3 (s, 1H) ; 8,1 (s, 1H) ; 6,8 (s, 2H).

### 5.2 acide 5-trifluorométhyl-1H-pyrrolo[2,3-b]pyridine-2-carboxylique

Dans un tube scellé de 25 mL, muni d'un agitateur magnétique et maintenu sous bullage d'argon, sont introduits 2 g (6,94 moles) de 2-amino-3-iodo-5-trifluorométhylpyridine obtenu selon le protocole décrit à l'étape 5.1, 1,89 g (20,83 mmoles) d'acide pyruvique, 2,41 g (20,83 mmoles) de 1,4-diazabicyclo[2.2.2]octane (DABCO) et 20 mL de diméthylformamide anhydre. Après quelques minutes, on ajoute 0,78 g (3,47 mmoles) d'acétate de palladium. Le mélange réactionnel est maintenu sous agitation et sous bullage d'argon pendant 20 minutes puis rapidement scellé et porté à 110 °C pendant 3h. La solution refroidie est concentrée sous pression réduite. Le résidu est repris par de l'acétate d'éthyle et de l'eau. Après décantation, on extrait la phase organique par deux fois 50 mL d'une solution aqueuse de soude 2N. Les phases aqueuses basiques sont rassemblées, refroidies à 0°C puis acidifiées par ajout d'acide chlorhydrique (pH 3). On extrait la phase aqueuse par de l'acétate d'éthyle (4x50 mL), les phases organiques rassemblées sont séchées sur sulfate de sodium puis concentrées sous pression réduite. On obtient 0,97 g de produit attendu sous forme de poudre jaune que l'on utilise tel quel dans les étapes suivantes.
R.M.N. ¹H (DMSO D₆), δ (ppm) : 12,8 (s, 1H) ; 8,7 (d, 1H) ; 8,5 (d, 1H) ; 7,2 (s, 1H).

### 5.3 5-trifluorométhyl-1H-pyrrolo[2,3-b]pyridine-2-carboxylate d'éthyle

Dans un ballon de 25 mL, muni d'un agitateur magnétique, est introduit 0,9 g (3,91 mmoles) de l'acide 5-trifluorométhyl-1*H*-pyrrolo[2,3-b]pyridine-2-carboxylique obtenu selon le protocole décrit à l'étape 5.2 et 10 mL d'éthanol. A cette solution, on ajoute 0,5 mL d'acide sulfurique concentré. Le mélange réactionnel est ensuite porté à reflux pendant 20 heures. La solution refroidie est concentrée sous pression réduite. Le résidu est repris au dichlorométhane (50 mL), on lave la phase organique successivement avec trois fois 20 mL d'une solution aqueuse saturée en hydrogénocarbonate de sodium, avec 20 mL d'eau puis avec 20 mL d'une solution aqueuse saturée en chlorure de sodium. On la sèche sur sulfate de sodium puis on la concentre sous pression réduite. On isole 0,85 g du produit attendu sous forme d'une poudre jaune.
R.M.N. ¹H (DMSO D₆), δ (ppm) : 13,1 (s, 1H) ; 8,8 (s, 1H) ; 8,6 (s, 1H) ; 7,3 (s, 1H) ; 4,4 (q, 2H) ; 1.35 (t, 3H).

5.4 5-trifluorométhyl-1-[(4-pyridyl)methyl]-1*H*-pyrrolo[2,3-*b*]pyridine-2-carboxylate d'éthyle A une solution de 1 g (3,87 mmoles) de produit obtenu selon le protocole décrit à l'étape 5.3, dans 20 mL de tétrahydrofurane sec, maintenue sous atmosphère inerte, sont ajoutés successivement sous agitation, 0,63 g (5,81 mmoles) de 4-pyridylméthanol puis 1,52 g (5,81 mmoles) de triphénylphosphine. On ajoute ensuite, goutte à goutte, 1,01 g (5,81 mmoles) d'azodicarboxylate de diéthyle. Le mélange réactionnel est alors agité pendant 20 h à température ambiante puis concentré sous pression réduite. On purifie l'huile résultante par chromatographie sur colonne de gel de silice en éluant avec un mélange d'heptane et d'acétate d'éthyle. On isole 1,23 g du produit attendu sous la forme d'un solide blanc.
R.M.N. ¹H (DMSO D₆), δ (ppm) : 8,8 (d, 1H) ; 8,7 (d, 1H) ; 8,45 (d, 2H) ; 7,55 (s, 1H) ; 6,95 (d, 2H) ; 5,9 (s, 2H) ; 4,3 (q, 2H) ; 1,2 (t, 3H).

### 5.5 N-(2-méthyl-benzothiazol-5-yl)-5-trifluorométhyl-1-[(4-pyridyl)méthyl]-1H-pyrrolo[2,3-b]pyridine-2-carboxamide (composé n°5)

Dans un tricol de 100 mL, refroidi à 0°C et muni d'une agitation magnétique, sont introduits sous balayage d'azote, 0,21 g (1,31 mmole) de 5-amino-2-méthylbenzothiazole et 5 mL de toluène sec. A cette solution, on ajoute ensuite lentement 0,82 mL (1,63 mole) d'une solution 2M de triméthylaluminium dans le toluène. Le mélange réactionnel obtenu est maintenu sous atmosphère d'azote et agité en laissant la température progressivement atteindre 70°C. A cette température, on ajoute, goutte à goutte en 5 minutes, une solution de 0,4 g (1,09 mmole) de 5-trifluorométhyl-1-[(4-pyridyl)methyl]-1*H*-pyrrolo[2,3-*b*]pyridine-2-carboxylate d'éthyle obtenu selon le protocole décrit à l'étape 5.4 dans 15 mL de toluène sec. Le mélange réactionnel est alors porté à reflux pendant 5 heures. A la solution refroidie à 0°C sont ensuite ajoutés 20 mL d'eau froide et 10 mL d'acide chlorhydrique 1N. Après 30 minutes d'agitation, le pH du mélange réactionnel est ajusté à 8 par ajout d'une solution saturée d'hydrogénocarbonate de sodium. On extrait ensuite ce mélange avec trois fois 50 mL d'acétate d'éthyle. Les phases organiques rassemblées sont lavées successivement avec 20 mL d'une solution saturée aqueuse d'hydrogénocarbonate de sodium, 20 mL d'eau puis avec 20 mL d'une solution saturée de chlorure de sodium. Après séchage sur sulfate de sodium, filtration et concentration sous pression réduite du filtrat organique, on purifie le solide jaune obtenu par chromatographies successives sur colonne de gel de silice. On isole 0,14 g de produit attendu sous la forme d'un solide blanc.
Point de fusion : 208 - 210 °C
R.M.N. ¹H (DMSO D₆), δ (ppm) : 10,8 (s, 1H) ; 8,8 (d, 2H) ; 8,45 (d, 2H) ; 8,3 (s, 1H) ; 7,95 (d, 1H); 7,7 (d, 1H) ; 7,65 (s, 1H) ; 7,0 (d, 2H) ; 6,0 (s, 2H) ; 2,8 (s, 3H).

### Exemple 6 (composé n°6)

### N-(2-méthyl-benzothiazol-5-yl)-1-(4-pyridyl)-1H-pyrrolo[2,3-b]pyridine-2-carboxamide

### 6.1 1-(4-pyridyl)-1H-pyrrolo[2,3-b]pyridine-2-carboxylate d'éthyle

Dans un tube scellé de 25 mL, muni d'un agitateur magnétique et maintenu sous bullage d'argon, sont introduits 0,2 g (1,05 mmole) de 1*H*-pyrrolo[2,3-*b*]pyridine-2-carboxylate d'éthyle obtenu selon le protocole décrit à l'étape 3.2, 0,244 g (1,16 mmole) de 4-iodopyridine, 0,01 g (0,05 mmole) d'iodure de cuivre, 0,47 g (2,21 mmoles) de phosphate tripotassique, 0,029 g (0,21 mmole) de *trans*-*N*,*N*'-diméthylcyclohexane-1,2-diamine racémique et 2 mL de toluène. Le mélange réactionnel est maintenu sous agitation et sous bullage d'argon pendant 20 minutes puis rapidement scellé et porté à 130 °C pendant 5 jours. La suspension refroidie est diluée dans 10 mL d'acétate d'éthyle et 20 mL d'eau. On extrait ensuite la phase aqueuse par deux fois 30 mL d'acétate d'éthyle. Les phases organiques rassemblées sont lavées successivement avec 10 mL d'une solution aqueuse saturée en hydrogénocarbonate de sodium, 10 mL d'eau et 10 mL d'une solution aqueuse saturée en chlorure de sodium. On sèche ensuite sur sulfate de sodium, on filtre et on concentre sous pression réduite. On isole 0,26 g de produit attendu sous forme d'une poudre marron.
R.M.N. ¹H (DMSO D₆), δ (ppm) : 8,75 (d, 2H) ; 8,4 (m, 1H) ; 8,25 (m, 1H) ; 7,5 (m, 3H) ; 7,3 (m, 1H) ; 4,2 (q, 2H) ; 1,2 (t, 3H).

### 6.2 N-(2-Méthyl-benzothiazol-5-yl)-1-(4-pyridyl)-1H-pyrrolo[2,3-b]pyridine-2-carboxamide

Dans un Keller de 25 mL muni d'une agitation magnétique, est introduit sous balayage d'argon, 0,147 g (0,9 mmole) de 5-amino-2-méthylbenzothiazole et 5 mL de toluène. On ajoute ensuite goutte à goutte, à 0°C, 0,56 mL (1,12 mmole) d'une solution 2M de triméthylaluminium dans le toluène. Le mélange réactionnel est porté à 70°C pendant 20 mn puis on ajoute 0,2 g (0,75 mmole) de 1-(4-pyridyl)-1*H*-pyrrolo[2,3-*b*]pyridine-2-carboxylate d'éthyle obtenu selon le protocole décrit à l'étape 6.1. Le mélange réactionnel est alors porté à reflux pendant 8 heures puis abondonné à température ambiante pendant le week end. A la solution refroidie à 0°C sont ensuite ajoutés 10 mL d'eau froide et 5 mL d'acide chlorhydrique 1N. Après 30 minutes d'agitation, le pH du mélange réactionnel est ajusté à 8 par ajout d'une solution saturée d'hydrogénocarbonate de sodium. On extrait ensuite ce mélange avec trois fois 20 mL d'acétate d'éthyle. Les phases organiques rassemblées sont lavées successivement avec 20 mL d'une solution saturée aqueuse d'hydrogénocarbonate de sodium, 20 mL d'eau puis avec 20 mL d'une solution saturée de chlorure de sodium. Après séchage sur sulfate de sodium, filtration et concentration sous pression réduite du filtrat organique, on purifie le solide orange obtenu par chromatographies successives sur colonne de gel de silice. Le solide isolé est ensuite lavé avec de l'éther isopropylique bouillant, puis séché sous pression réduite. On isole 0,217g de produit attendu sous la forme d'un solide orange.
Point de fusion : 236 - 238 °C
R.M.N. ¹H (DMSO D₆), δ (ppm) : 10,9 (s, 1H) 8,7 (d, 2H) ; 8,4 (m, 1H) ; 8,3 (m, 2H) ; 7,95 (d, 1H) ; 7,7 (dd, 1H) ; 7,55 (s, 1H) ; 7,5 (m, 2H) ; 7,3 (m, 1H) ; 2,8 (s, 3H).

### Exemple 7 (composé n°7)

### N-(2-méthyl-benzothiazol-5-yl)-1-(3-pyridyl)-1H-pyrrolo[2,3-b]pyridine-2-carboxamide

### 7.1 1-(3-pyridyl)-1H-pyrrolo[2,3-b]pyridine-2-carboxylate d'éthyle

Ce composé a été préparé selon le protocole expérimental décrit dans l'exemple 6.1 à partir du 1*H*-pyrrolo[2,3-b]pyridine-2-carboxylate d'éthyle obtenu selon le protocole décrit à l'étape 3.2 et de la 3-iodopyridine. On obtient un solide jaune.
R.M.N. ¹H (DMSO D₆), δ (ppm) : 8,65 (m, 2H) ; 8,4 (m, 1H) ; 8,25 (dd, 1H) ; 7,9 (m, 1H) ; 7,55 (m, 1H) ; 7,5 (s, 1H) ; 7,3 (m, 1H) ; 4,2 (q, 2H) ; 1,2 (t, 3H).

### 7.2 N-(2-méthyl-benzothiazol-5-yl)-1-(3-pyridyl)-1H-pyrrolo[2,3-b]pyridine-2-carboxamide (composé n°7)

Ce composé a été préparé selon le protocole expérimental décrit dans l'exemple 6.2 à partir du 1-(3-pyridyl)-1*H*-pyrrolo[2,3-*b*]pyridine-2-carboxylate d'éthyle obtenu selon le protocole décrit dans l'étape 7.1 et du 5-amino-2-méthylbenzothiazole. On obtient un solide jaune.
Point de fusion : 246 - 248 °C
R.M.N. ¹H (DMSO D₆), δ (ppm) : 11,2 (s, 1H) ; 8,7 (d, 1H) ; 8,6 (d, 1H) ; 8,4 (m, 1H) ; 8,3 (m, 2H) ; 7,9 (m, 2H) ; 7,7 (m, 1H) ; 7,55 (m, 2H) ; 7,3 (dd, 1H) ; 2,8 (s, 3H).

### Exemple 8 (composé n°8)

### N-(2-méthyl-benzothiazol-5-yl)-1-(2-pyridyl)-1H-pyrrolo[2,3-b]pyridine-2-carboxamide

### 8.1 1-(2-pyridyl)-1H-pyrrolo[2,3-b]pyridine-2-carboxylate d'éthyle

Ce composé a été préparé selon le protocole expérimental décrit dans l'exemple 6.1 à partir du 1*H*-pyrrolo[2,3-*b*]pyridine-2-carboxylate d'éthyle obtenu selon le protocole décrit à l'étape 3.2 et de la 2-iodopyridine. On obtient un solide beige.
R.M.N. ¹H (DMSO D₆), δ (ppm) : 8,55 (m, 1H) ; 8,4 (dd, 1H) ; 8,2 (dd, 1H) ; 8,1 (td, 1H) ; 7,8 (d, 1H) ; 7,5 (ddd, 1H) ; 7,4 (s, 1H) ; 7,3 (dd, 1H) ; 4,15 (q, 2H) ; 1,05 (s, 3H).

### 8.2 N-(2-méthyl-benzothiazol-5-yl)-1-(2-pyridyl)-1H-pyrrolo[2,3-b]pyridine-2-carboxamide (compose n°8)

Ce composé a été préparé selon le protocole expérimental décrit dans l'exemple 6.2 à partir du 1-(2-pyridyl)-1*H*-pyrrolo[2,3-*b*]pyridine-2-carboxylate d'éthyle obtenu selon le protocole décrit à l'étape 8.1 et du 5-amino-2-méthylbenzothiazole. On obtient un solide blanc.
Point de fusion : 112 - 114 °C
R.M.N. ¹H (DMSO D₆), δ (ppm) : 10,8 (s, 1H) ; 8,4 (m, 2H) ; 8,25 (m, 2H) ; 8,05 (m, 1H) 7,95 (m, 2H) ; 7,65 (dd, 1H) ; 7,35 (m, 3H) ; 2,8 (s, 3H).

Le tableau 1 qui suit illustre les structures chimiques et les propriétés physiques de quelques composés de formule générale (I) selon l'invention dans laquelle le noyau pyrrolopyridine est une pyrrolo[2,3-b]pyridine éventuellement substituée.

Dans ce tableau :
la colonne « PF » renseigne les points de fusion des produits en degrés Celsius (°C) ;

**Tableau 1**

| | | | | | |
|---|---|---|---|---|---|
| 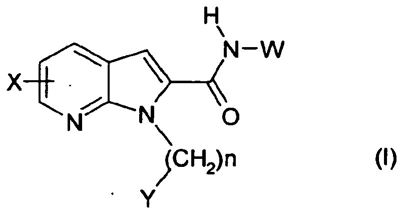 | | | | | |

| **N°** | **X** | **n** | **Y** | **W** | **PF (°C)** |
|---|---|---|---|---|---|
| 1 | 5-F | 1 | 4-pyridinyl- | 2-méthyl-benzothiazol-5-yle | 217-219 |
| 2 | 5-F | 1 | 4-pyridinyl- | 1,2-diméthyl-benzimidazol-5-yle | 263-265 |
| 3 | H | 1 | 4-pyridinyl- | 1,2-diméthyl-benzimidazol-5-yle | 236-246 |
| 4 | H | 1 | 4-pyridinyl- | 2-méthyl-benzothiazol-5-yle | 211-236 |
| 5 | 5-CF₃ | 1 | 4-pyridinyl- | 2-méthyl-benzothiazol-5-yle | 208-210 |
| 6 | H | 0 | 4-pyridinyl- | 2-méthyl-benzothiazol-5-yle | 236-238 |
| 7 | H | 0 | 3-pyridinyl- | 2-méthyl-benzothiazol-5-yle | 246-248 |
| 8 | H | 0 | 2-pyridinyl- | 2-méthyl-benzothiazol-5-yle | 112-114 |

Les composés de l'invention ont été soumis à des essais pharmacologiques *in vitro* et *in vivo* qui ont mis en évidence leur intérêt comme substances à activités thérapeutiques.

### Test d'inhibition du courant induit par la capsaïcine sur les DRG de rat

### - Culture primaire de cellules de ganglions de racine dorsale (DRG) de rat :

Les neurones du DRG expriment naturellement le récepteur TRPV1.

Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCI, 2 mM glutamine, 100 µg/mL gentamicine et 50 ng/mL de NGF, puis déposées sur des lamelles de verre recouvertes de laminine (0.25 x 10⁶ cellules par lamelle) qui sont ensuite placées dans des boîtes 12 puits Corning. Les cellules sont incubées à 37°C en atmosphère humidifiée contenant 5% de CO₂ et 95% d'air. De la cytosine β-D-arabinoside (1 µM) est ajoutée 48 h après la mise en culture, pour prévenir le développement des cellules non neuronales. Les lamelles sont transférées dans les chambres expérimentales pour les études de patch-clamp après 7-10 jours de culture.

### - Electrophysiologie :

Les chambres de mesure (volume 800 µl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 mL/min) à l'aide d'un distributeur de solutions acceptant 8 encrées et dont la sortie unique, constituée par un tube de polyéthylène (ouverture 500µm) est placée à moins de 3 mm de la cellule étudiée. La configuration "cellule entière" de la technique de patch-clamp a été utilisée.

Les pipettes en verre borosilicaté (résistance 5-10 MOhms) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1D (Axon Instruments, Foster city, Califomie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.

L'application d'une solution de capsaïcine 1 µM, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.

Les pourcentages d'inhibition de la réponse capsaïcine (1 µM) sont compris entre 20% et 100% pour les composés les plus actifs de l'invention testés à des concentrations de 0,1 à 10 nM (voir l'exemple du tableau 2).

**Tableau 2**

| N° composé | % inhibition en patch DRG |
|---|---|
| Composé 1 | 50 % (10 nM) |

Les composés de l'invention sont donc des antagonistes efficaces *in vitro* des récepteurs de type TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formule (I), ou un sel pharmaceutiquement acceptable, ou encore un hydrate ou un solvat dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou iatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.

Les composés de l'invention peuvent également être utilisés pour prévenir et/ou traiter les désordres métaboliques tels que le diabète.

Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.

On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.

De même, les produits de la présente invention peuvent être utiles dans la prévention et/ou le traitement des désordres respiratoires tels que l'asthme, la toux, la pneumopathie obstructive chronique ou COPD (chronic obstructive pulmonary disease), la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour traiter la dépression.

Les composés de l'invention peuvent aussi être utilisés pour traiter les maladies du système nerveux central telles que la sclérose en plaques.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple; une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I) 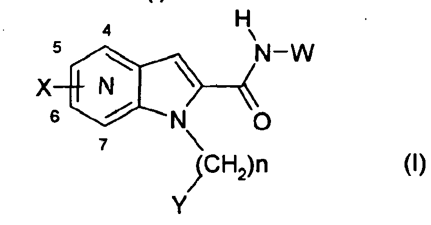 n est égal à 0, 1, 2 ou 3 ;
le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et / ou 7 par un ou plusieurs substituants X, identiques ou différents l'un de l'autre, choisis parmi un atome d'halogène ou un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₃-alkylène-O-, C₁-C₆-fluoroalcoxyle, cyano, C(O)NR₁R₂, nitro, NR₁R₂, C₁-C₆-thioalkyle, -S(O)-C₁-C₆-alkyle, -S(O)₂-C₁-C₆-alkyle, SO₂NR₁R₂, NR₃COR₄, NR₃SO₂R₅, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₆-cycloalkyle, C₃-C₇-cycloalkyle-C₁-C₃-alkylène-O-, C₁-C₆-fluoroalkyle, C₁-C₆-alcoxyle, C₃-C₇-cycloalcoxyle, C₃-C₇-cycloalkyle-C₁-C₃-alkylène-O-, C₁-C₆-fluoroalcoxyle, nitro ou cyano ;
Y représente un hétéroaryle éventuellement substitué par un ou plusieurs groupes choisis parmi un atome d'halogène ou un groupe C₁-C₈-alkyle, C₃-C₇-cycloalkyle, C₃-C₇-cycloalkyle-C₁-C₃-alkylène, C₁-C₆-fluoroalkyle, hydroxyle, C₁-C₈-alcoxyle, C₃-C₇-cycloalcoxyle, C₃-C₈-cycloalkyle-C₁-C₆-alkylène-O-, C₁-C₆-fluoroalcoxyle, cyano, C(O)NR₁R₂, nitro, NR₁R₂, C₁-C₆-thioalkyle, C₁-C₆-thiofluoroalkyle, thiol, -S(O)-C₁-C₆-alkyle, -S(O)-C₃-C₇-cycloalkyle, -S(O)-C₁-C₆-alkylène-C₃-C₇-cycloalkyle, -S(O)₂-C₁-C₆-alkyle, -S(O)₂-C₃-C₇-cycloalkyle, -S(O)₂-C₁-C₆-alkylène-C₃-C₇-cycloalkyle SO₂NR₁R₂, NR₃COR₄, NR₃SO₂R₅, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
R₁ et R₂, représentent, indépendamment l'un de l'autre, un atome d'hydrogène ou un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₈-alkylène-O-, C₁-C₆-fluoroalcoxyle, nitro ou cyano ;
ou R₁ et R₂ forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe C₁-C₆-alkyle, C₃-C₇-cycloalkyle, C₃-C₇-cycloalkyle-C₁**-**C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogéné, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
R₃ et R₄ représentent, indépendamment l'un de l'autre, un atome d'hydrogène ou un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
R₅ représente un groupe C₁-C₆-alkyle, C₃-C₇-cycloalkyle, C₃-C₇-cycloalkyle-C₁-C₃-alkylène, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkylène-O-, C₁-C₆-fluoroalcoxyle, nitro ou cyano ;
W représente un groupe bicyclique fusionné de formule : 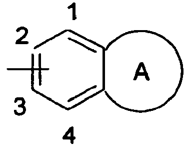 lié à l'atome d'azote par les positions 1, 2, 3 ou 4 ;
A représente un hétérocycle de 5 à 7 chaînons comprenant de un à trois hétéroatomes choisis parmi O, S ou N ;
le ou les atomes de carbone de A étant éventuellement substitués par un ou plusieurs groupes choisis parmi un atome d'hydrogène ou un groupe C₁-C₆-alkyle, C₃-C₇-cycloalkyle, C₃-C₇-cycloalkyle-C₁-C₃-alkylène, C₁-C₆-fluoroalkyle, aryle, aryl-C₁-C₅-alkylène, hétéroaryle ou hétéroaryl-C₁-C₅-alkylène, oxo ou thio; l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cydoalkyle-C₁-C₆-alkylène-O-, C₁-C₆-fluoroalcoxyle, nitro ou cyano ;
le ou les atomes d'azote de A étant éventuellement substitués par R₆ lorsque l'azote est adjacent à un atome de carbone substitué par un groupe oxo, ou par R₇ dans les autres cas ;
R₆ représente un atome d'hydrogène ou un groupe C₁-C₆-alkyle, C₃-C₇-cydoalkyle, C₃-C₇-cycloalkyle-C₁-C₃-alkylène, C₁-C₆-fluoroalkyle, aryle, aryl-C₁-C₅-alkylène, hétéroaryle ou hétéroaryl-C₁-C₅-alkylène, l'aryle ou l'hétéroaryle étant éventuellement substitués par un ou plusieurs substituants choisis parmi un halogène, un groupe C₁-C₆-alkyle, C₃-C₇-cycloalkyle, C₃-C₇-cycloalkyle-C₁-C₃-alkylène, C₁-C₆-fluoroalkyle, C₁-C₆-alcoxyle, C₃-C₇-cycloalcoxyle, C₃-C₇-cycloalkyle-C₁-C₆-alkyléne-O-, C₁-C₆-fluoroalcoxyle, nitro ou cyano ;
R₇ représente un atome d'hydrogène ou un groupe C₁-C₆-alkyle, C₃-C₇-cycloalkyle, C₃-C₇-cycloalkyle-C₁-C₃-alkylène, C₁-C₆-fluoroalkyle, aryle-C₁-C₆-alkylène, C₁-C₅-alkyle-C(O)-, C₃-C₇-cycloalkyle-C₁-C₃-alkylène-(CO)-, C₁-C₆-fluoroalkyle-C(O)-, C₃-C₇-cycloalkyle-C(O)-, aryle-C(O)-, aryle-C₁-C₆-alkylène-C(O)-, C₁-C₆-alkyle-S(O)₂-, C₁-C₆-fluoroalkyle-S(O)₂-, C₃-C₇-cycloalkyle-S(O)₂-, C₃-C₇-cycloalkyle-C₁-C₃-alkylène-S(O)₂-, aryle-S(O)₂- ou aryle-C₁-C₆-alkylène-S(O)₂- ou aryle ;
le ou les atomes de soufre de l'hétérocycle A pouvant être sous forme oxydée ;
le ou les atomes d'azote de l'hétérocycle A pouvant être sous forme oxydée ;
l'atome d'azote en position 4, 5, 6 ou 7 de la pyrrolopyridine peut être sous forme oxydée ;
à l'état de base ou de sel d'addition à un acide, ainsi qu'à l'état d'hydrate ou de solvat.

2. Composé de formule (I) selon la revendication 1, **caractérisé en ce que** n est égal à 0 ou 1 ; à l'état de base ou de sel d'addition à un acide, ainsi qu'à l'état d'hydrate ou de solvat.

3. Composé de formule (I) selon la revendication 1 ou 2, **caractérisé en ce que** le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et / ou 7 par un ou plusieurs substituants X, identiques ou différents l'un de l'autre, choisis parmi un atome d'halogène ou un groupe C₁-C₆-alkyle, C₁-C₆-fluoroalkyle ; à l'état de base ou de sel d'addition à un acide, ainsi qu'à l'état d'hydrate ou de solvat.

4. Composé de formule (I) selon la revendication 1 ou 2, **caractérisé en ce que** le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 5 par un substituant X tel que défini dans la formule générale (I) selon la revendication 1 ; à l'état de base ou de sel d'addition à un acide, ainsi qu'à l'état d'hydrate ou de solvat.

5. Composé de formule (I) selon l'une quelconque des revendications 1 à 3, **caractérisé en ce que** le noyau pyrrolopyridine est un groupe pyrrolo[2,3-*b*]pyridine éventuellement substitué en position carbonée 4, 5, 6 et / ou 7 par un ou plusieurs substituants X, identiques ou différents l'un de l'autre, choisis parmi un atome d'halogène ou un groupe C₁-C₆-fluoroalkyle ; à l'état de base ou de sel d'addition à un acide, ainsi qu'à l'état d'hydrate ou de solvat.

6. Composé de formule (I) selon la revendication 5, **caractérisé en ce que** le noyau pyrrolopyridine est un groupe pyrrolo[2,3-*b*]pyridine éventuellement substitué en position carbonée 5 par un substituant X choisi parmi un atome d'halogène ou un groupe C₁-C₆-fluoroalkyle ; à l'état de base ou de sel d'addition à un acide, ainsi qu'à l'état d'hydrate ou de solvat.

7. Composé de formule (I) selon l'une quelconque des revendications 1 à 6, **caractérisé en ce que** Y représente un groupe choisi parmi les groupes imidazolyle, thiazolyle, oxazolyle, furanyle, thiophényle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle, quinoxalinyle, cinnolinyle, quinazolinyle, phtalazinyle, naphtyridinyle ; ce groupe étant éventuellement substitué comme défini dans la formule générale (I) selon la revendication 1 ; à l'état de base ou de sel d'addition à un acide, ainsi qu'à l'état d'hydrate ou de solvat.

8. Composé de formule (I) selon la revendication 7, **caractérisé en ce que** Y représente un pyridinyle ; à l'état de base ou de sel d'addition à un acide, ainsi qu'à l'état d'hydrate ou de solvat.

9. Composé de formule (I) selon l'une quelconque des revendications 1 à 8, **caractérisé en ce que** W est choisi parmi les groupes indolinyle, isoindolinyle, indolyle, isoindolyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle,
dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépin-yle, tétrahydrobenzo[*e*][1,4]-diazépinyle, tétrahydrobenzo[*b*][1,4]oxazépinyle ou tétrahydrobenzo[*b*][1,4]thiazépinyle ; ces groupes pouvant être éventuellement substitués comme défini dans la formule générale (I) selon la revendication 1 ; à l'état de base ou de sel d'addition à un acide, ainsi qu'à l'état d'hydrate ou de solvat.

10. Composé de formule (I) selon la revendication 9, **caractérisé en ce que** W représente un groupe benzimidazolyle ou benzothiazolyle, le ou les atomes de carbones de W étant éventuellement substitués par un ou plusieurs groupes C₁-C₆-alkyle ; le ou les atomes d'azote de W étant éventuellement substitués un groupe C₁-C₆-alkyle ; à l'état de base ou de sel d'addition à un acide, ainsi qu'à l'état d'hydrate ou de solvat.

11. Procédé de préparation d'un composé de formule (I) selon l'une quelconque des revendications 1 à 10, **caractérisé en ce que** l'on fait réagir un composé de formule générale (IV) 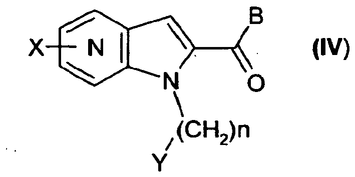 dans laquelle n, X et Y sont tels que définis dans la formule générale (I) selon la revendication 1 et B représente un groupe C₁-C₄-alcoxyle,
avec un amidure du composé de formule générale (V) 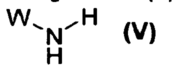 dans laquelle W est tel que défini dans la formule générale (I) selon la revendication 1, au reflux d'un solvant, l'amidure du composé de formule générale (V) étant préparé par action préalable du triméthylaluminium sur les composés de formule générale (V).

12. Procédé de préparation d'un composé de formule (I) selon l'une quelconque des revendications 1 à 10, **caractérisé en ce que** l'on transforme un composé de formule générale (IV) 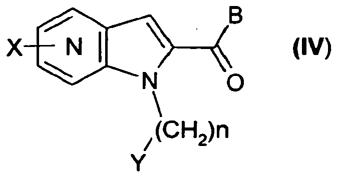 dans laquelle n, X et Y sont tels que définis dans la formule générale (I) selon la revendication 1 et B représente un groupe hydroxyle,
en chlorure d'acide par action du chlorure de thionyle au reflux d'un solvant,
puis **en ce que** l'on fait réagir, en présence d'une base, le composé de formule générale (IV) obtenu, dans laquelle sont tels que définis dans la formule générale (I) selon la revendication 1 et B représente un atome de chlore, avec le composé de formule générale (V), 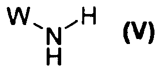 dans laquelle W est tel que défini dans la formule générale (I) selon la revendication 1 ; ou bien **en ce que** l'on effectue une réaction de couplage entre un composé de formule générale (IV) dans laquelle n, X et Y sont tels que définis dans la formule générale (I) selon la revendication 1 et B représente un groupe hydroxyle,
et le composé de formule générale (V), dans laquelle W est tel que défini dans la formule générale (I) selon la revendication 1, en présence d'un agent de couplage et d'une base, dans un solvant.

13. Médicament, **caractérisé en ce qu'**il comprend un composé de formule (I), selon l'une quelconque des revendications 1 à 10, ou un sel pharmaceutiquement acceptable, ou encore un hydrate ou un solvat du composé de formule (I).

14. Composition pharmaceutique, **caractérisée en ce qu'**elle comprend un composé de formule (I), selon l'une quelconque des revendications 1 à 10, ou un sel pharmaceutiquement acceptable, un hydrate ou un solvat de ce composé, ainsi qu'au moins un excipient pharmaceutiquement acceptable.

15. Utilisation d'un composé de formule (I), selon l'une quelconque des revendications 1 à 10, pour la préparation d'un médicament destiné à prévenir ou à traiter les pathologies dans lesquelles les récepteurs de type TRPV1 sont impliqués.

16. Utilisation d'un composé de formule (I), selon l'une quelconque des revendications 1 à 10, pour la préparation d'un médicament destiné à prévenir ou à traiter douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques et la dépression.

## Claims

1. Compound corresponding to the formula (I): 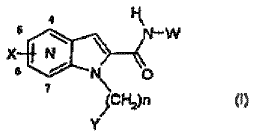 in which:
n is equal to 0, 1, 2 or 3;
the pyrrolopyridine ring system is a pyrrolo[3,2-b]pyridine group, a pyrrolo[3,2-c]pyridine group, a pyrrolo[2,3-c]pyridine group or a pyrrolo[2,3-b]pyridine group;
the pyrrolopyridine ring system optionally being substituted in the 4, 5, 6 and/or 7 carbon-based position by one or more substituents X, which are identical to or different from one another, chosen from a halogen atom or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₃-alkylene-O-, C₁-C₆-fluoroalkoxyl, cyano, C(O)NR₁R₂, nitro, NR₁R₂, C₁-C₆-thioalkyl, -S(O)-C₁-C₆-alkyl, -S(O)₂-C₁-C₆-alkyl , SO₂NR₁R₂, NR₃COR₄, NR₃SO₂R₅, aryl, aryl-C₁-C₅-alkylene, heteroaryl or heteroaryl-C₁-C₅-alkylene group,
the aryl or heteroaryl optionally being substituted by one or more substituents chosen from a halogen or a C₁-C₆-alkyl, C₃-C₇-cycloalkyl, C₃-C₇-cycloalkyl-C₁-C₃-alkylene-O-, C₁-C₆-fluoroalkyl, C₁-C₆-alkoxyl, C₃-C₇-cycloalkoxyl, C₃-C₇-cycloalkyl-C₁-C₃-alkylene-O-, C₁-C₆-fluoroalkoxyl, nitro or cyano group;
Y represents a heteroaryl optionally substituted by one or more groups chosen from a halogen atom or a C₁-C₆-alkyl, C₃-C₇-cycloalkyl, C₃-C₇-cycloalkyl-C₁-C₃-alkylene, C₁-C₆-fluoroalkyl, hydroxyl, C₁-C₆-alkoxyl, C₃-C₇-cycloalkoxyl, C₃-C₇-cycloalkyl-C₁-C₆-alkylene-O-, C₁-C₆-fluoroalkoxyl, cyano, C(O)NR₁R₂, nitro, NR₁R₂, C₁-C₆-thioalkyl, C₁-C₆-thiofluoroalkyl, thiol, -S(O)-C₁-C₆-alkyl, -S(O)-C₃-C₇-cycloalkyl, -S(O)-C₁-C₆-alkylene-C₃-C₇-cycloalkyl, -S(O)₂-C₁-C₆-alkyl, -S(O)₂-C₃-C₇-cycloalkyl, -S(O)₂-C₁-C₆-alkylene-C₃-C₇-cycloalkyl, SO₂NR₁R₂, NR₃COR₄, NR₃SO₂R₅, aryl, aryl-C₁-C₅-alkylene, heteroaryl or heteroaryl-C₁-C₅-alkylene group, the aryl or heteroaryl optionally being substituted by one or more substituents chosen from a halogen or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano groups;
R₁ and R₂ represent, independent of one another, a hydrogen atom or a C₁-C₆-alkyl, C₃-C₇-cycloalkyl, C₃-C₇-cycloalkyl-C₁-C₃-alkylene, aryl, aryl-C₁-C₅-alkylene, heteroaryl or heteroaryl-C₁-C₅-alkylene group, the aryl or the heteroaryl optionally being substituted by one or more substituents chosen from a halogen or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano group;
or R₁ and R₂ together form, with the nitrogen atom which carries them, an azetidine, pyrrolidine, piperidine, azepine, morpholine, thiomorpholine, piperazine or homopiperazine group, this group optionally being substituted by a C₁-C₆-alkyl, C₃-C₇-cycloalkyl, C₃-C₇-cycloalkyl-C₁-C₃-alkylene, aryl, aryl-C₁-C₅-alkylene, heteroaryl or heteroaryl-C₁-C₅-alkylene group, the aryl or the heteroaryl optionally being substituted by one or more substituents chosen from a halogen or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano group;
R₃ and R₄ represent, independently of one another, a hydrogen atom or a C₁-C₆-alkyl, C₃-C-₇-cycloalkyl, C₃-C₇-cycloalkyl-C₁-C₃-alkylene, aryl, aryl-C₁-C₅-alkylene, heteroaryl or heteroaryl-C₁-C₅-alkylene group, the aryl or heteroaryl optionally being substituted by one or more substituents chosen from a halogen atom or a C₁-C₆-alkyl, C3-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano group;
R₅ represents a C₁-C₆-alkyl, C₃-C₇-cycloalkyl, C₃-C₇-cycloalkyl-C₁-C₃-alkylene, aryl, aryl-C₁-C₅-alkylene, heteroaryl or heteroaryl-C₁-C₅-alkylene group, the aryl or the heteroaryl optionally being substituted by one or more substituents chosen from a halogen or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano group;
W represents a fused bicyclic group of formula: 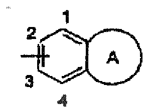
bonded to the nitrogen atom via the positions 1, 2, 3 or 4 ;
A represents a 5- to 7-membered heterocycle comprising one to three heteroatoms chosen from O, S or N;
the carbon atom or atoms of A optionally being substituted by one or more groups chosen from a hydrogen atom or a C₁-C₆-alkyl, C₃-C₇-cycloalkyl, C₃-C₇-cycloalkyl-C₁-C₃-alkylene, C₁-C₆-fluoroalkyl, aryl, aryl-C₁-C₅-alkylene, heteroaryl or heteroaryl-C₁-C₅-alkylene, oxo or thio group; the aryl or the heteroaryl optionally being substituted by one or more substituents chosen from a halogen or a C₁-C₆-alkyl, C₃-C₇-cycloalkyl, C₃ - C₇-cycloalkyl-C₁-C₃ - alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano group;
the nitrogen atom or atoms of A optionally being substituted by R₆, when the nitrogen is adjacent to a carbon atom substituted by an oxo group, or by R₇ in the other cases;
R₆ represents a hydrogen atom or a C₁-C₆-alkyl, C₃-C₇-cycloalkyl, C₃-C₇-cycloalkyl-C₁-C₃-alkylene, C₁-C₆-fluoroalkyl, aryl, aryl-C₁-C₆-alkylene, heteroaryl or heteroaryl-C₁-C₅-alkylene group, the aryl or the heteroaryl optionally being substituted by one or more substituents chosen from a halogen or a C₁-C₆-alkyl, C₃-C₇-cycloalkyl, C₃-C₇-cycloalkyl-C₁-C₃-alkylene, C₁-C₆-fluoroalkyl, C₁-C₆-alkoxyl, C₃-C₇-cycloalkoxyl, C₃-C₇-cycloalkyl-C₁-C₆-alkylene-O-, C₁-C₆-fluoroalkoxyl, nitro or cyano group;
R₇ represents a hydrogen atom or a C₁-C₆-alkyl, C₃-C₇-cycloalkyl, C₃-C₇-cycloalkyl-C₁-C₃-alkylene, C₁-C₆-fluoroalkyl, aryl-C₁-C₆-alkylene, C₁-C₆-alkyl-C(O)-, C₃-C₇-cycloalkyl-C₁-C₃-alkylene-(CO) -, C₁-C₆-fluoroalkyl-C(O)-, C₃-C₇-cycloalkyl-C(O)-, aryl-C (O) -, aryl-C₁-C₆-alkylene-C(O)-, C₁-C₆-alkyl-S(O)₂-, C₁-C₆-fluoroalkyl-S(O)₂-, C₃-C₇-cycloalkyl-S(O)₂-, C₃-C₇-cycloalkyl-C₁-C₃-alkylene-S(O)₂-, aryl-S(O)₂-, aryl-C₁-C₆-alkylene-S(O)₂- or aryl group;
it being possible for the sulfur atom or atoms of the heterocycle A to be in the oxidized form;
it being possible for the nitrogen atom or atoms of the heterocycle A to be in the oxidized form;
the nitrogen atom in the 4, 5, 6 or 7 position of the pyrrolopyridine can be in the oxidized form;
in the base form or in the form of an addition salt with an acid, and also in the hydrate or solvate form.

2. Compound of formula (I) according to Claim 1, **characterized in that** n is equal to 0 or 1; in the base form or in the form of an addition salt with an acid, and also in the hydrate or solvate form.

3. Compound of formula (I) according to Claim 1 or 2, **characterized in that** the pyrrolopyridine ring system is a pyrrolo [3,2-*b*] pyridine group, a pyrrolo[3,2-*c*] pyridine group, a pyrrolo[2,3-*c*]pyridine group or a pyrrolo[2,3-*b*]pyridine group;
the pyrrolopyridine ring system optionally being substituted in the 4, 5, 6 and/or 7 carbon-basted position by one or more substituents X, which are identical to or different from one another, chosen from a halogen atom or a C₁-C₆-alkyl or C₁-C₆-fluoroalkyl group; in the base form or in the form of an addition salt with an acid, and also in the hydrate or solvate form.

4. Compound of formula (I) according to Claim 1, or 2, **characterized in that** the pyrrolopyridine ring system is a pyrrolo[3,2-*b*]pyridine group, a pyrrolo[3,2-*c*] pyridine group, a pyrrolo[2,3-*c*]pyridine group or a pyrrolo[2,3-*b*]pyridine group;
the pyrrolopyridine ring system optionally being substituted in the 5 carbon-based position by a substituent X as defined in the general formula (I) according to Claim 1; in the base form or in the form of an addition salt with an acid, and also in the hydrate or solvate form.

5. Compound of formula (I) according to any one of Claims 1 to 3, **characterized in that** the pyrrolopyridine ring system is a pyrrolo[2,3-*b*]pyridine group optionally substituted in the 4, 5, 6 and/or 7 carbon-based position by one or more substituents X, which are identical to or different from one another, chosen from a halogen atom or a C₁-C₆-fluoroalkyl group; in the base form or in the form of an addition salt with an acid, and also in the hydrate or solvate form.

6. Compound of formula (I) according to Claim 5, **characterized in that** the pyrrolopyridine ring system is a pyrrolo[2,3-b]pyridine group optionally substituted in the 5 carbon-base position by a substituent X chosen from a halogen atom or a C₁-C₆-fluoroalkyl group; in the base form or in the form of an addition salt with an acid, and also in the hydrate or solvate form.

7. Compound of formula (I) according to any one of Claims 1 to 6, **characterized in that** Y represents a group chosen from the imidazolyl, thiazolyl, oxazolyl, furanyl, thiophenyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl, isoquinolinyl, quinoxalinyl, cinnolinyl, quinazolinyl, phthalazinyl or naphthyridinyl groups; this group optionally being substituted as defined in the general formula (I) according to Claim 1; in the base form or in the form of an addition salt with an acid, and also in the hydrate or solvate form.

8. Compound of formula (I) according to Claim 7, **characterized in that** Y represents a pyridinyl; in the base form or in the form of an addition salt with an acid, and also in the hydrate or solvate form.

9. Compound of formula (I) according to any one of Claims 1 to 8, **characterized in that** W is chosen from the indolinyl, isoindolinyl, indolyl, isoindolyl, benzofuranyl, dihydrobenzofuranyl, benzothiophenyl, dihydrobenzothiophenyl, benzoxazolyl, dihydrobenzoxazolinyl, isobenzofuranyl, dihydroisobenzofuranyl, benzimidazolyl, dihydrobenzimidazolyl, indazolyl, benzothiazolyl, isobenzothiazolyl, dihydroisobenzothiazolyl, benzotriazolyl, quinolinyl, dihydroquinolinyl, tetrahydroquinolinyl, isoquinolinyl, dihydroisoquinolinyl, tetrahydroisoquinolinyl, benzoxazinyl, dihydrobenzoxazinyl, benzothiazinyl, dihydrobenzothiazinyl, cinnolinyl, quinazolinyl, dihydroquinazolinyl, tetrahydroquinazalinyl, quinoxalinyl, dihydroquinoxalinyl, tetrahydroquinoxalinyl, phthalazinyl, dihydrophthalazinyl, tetrahydrophthalazinyl, tetrahydrobenz[*b*]azepinyl, tetrahydrobenz[*c*]azepinyl, tetrahydrobenz[*d*]azepinyl, tetrahydrabenzo[*b*][1,4]diazepinyl, tetrahydrobenzo[*e*][1,4]-diazepinyl, tetrahydrobenzo[*b*][1,4]oxazepinyl or tetra-hydrobenzo[*b*][1,4]thiazepinyl groups;
it being possible for these groups to be optionally substituted as defined in the general formula (I) according to Claim 1; in the base form or in the form of an addition salt with an acid, and also in the hydrate or solvate form.

10. Compound of formula (I) according to Claim 9, **characterized in that** W represents a benzimidazolyl or benzothiazolyl group, the carbon atom or atoms of W optionally being substituted by one or more C₁-C₆-alkyl groups and the nitrogen atom or atoms of W optionally being substituted by a C₁-C₆-alkyl group; in the base form or in the form of an addition salt with an acid, and also in the hydrate or solvate form.

11. Process for the preparation of a compound of formula (I) according to any one of Claims 1 to 10, **characterized in that** a compound of general formula (IV) : 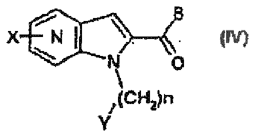 in which n, X and Y are as defined in the general formula (I) according to Claim 1 and B represents a C₁-C₄-alkoxyl group,
is reacted with an amide of the compound of general formula (V): 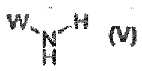 in which W is as defined in the general formula (I) according to Claim 1,
at reflux of a solvent, the amide of the compound of general formula (V) being prepared by the prior action of trimethylaluminium on the compounds of general formula (V).

12. Process for the preparation of a compound of formula (I) according to any one of Claims 1 to 10, **characterized in that** a compound of general formula (IV) : 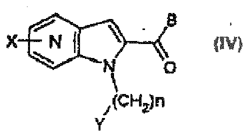 in which n, X and Y are as defined in the general formula (I) according to Claim 1 and B represents a hydroxyl group,
is converted to the acid chloride by the action of thionyl chloride at reflux of a solvent,
and then **in that** the compound of general formula (IV) obtained, in which they are as defined in the general formula (I) according to Claim 1 and B represents a chlorine atom, is reacted, in the presence of a base, with the compound of general formula (V): 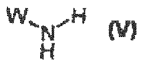 in which W is as defined in the general formula (I) according to Claim 1;
or else **in that** a coupling reaction is carried out between a compound of general formula (IV), in which n, X and Y are as defined in the general formula (I) according to Claim 1 and B represents a hydroxyl group, and the compound of general formula (V), in which W is as defined in the general formula (I) according to Claim 1, in the presence of a coupling agent and of a base in a solvent.

13. Medicament, **characterized in that** it comprises a compound of formula (I) according to any one of Claims 1 to 10 or a pharmaceutically acceptable salt or also a hydrate or a solvate of the compound of formula (I).

14. Pharmaceutical composition, **characterized in that** it comprises a compound of formula (I) according to any one of Claims 1 to 10 or a pharmaceutically acceptable salt, a hydrate or a solvate of this compound, and also at least one pharmaceutically acceptable excipient.

15. Use of a compound of formula (I) according to any one of Claims 1 to 10 in the preparation of a medicament intended to prevent or treat the pathologies in which receptors of TRPV1 type are involved.

16. Use of a compound of formula (I) according to any one of Claims 1 to 10 in the preparation of a medicament intended to prevent or treat pain, inflammation, metabolic disorders, urological disorders, gynaecological disorders, gastrointestinal disorders, respiratory disorders, psoriasis, pruritis, irritation of the skin, eyes or mucous membranes, herpes, shingles, multiple sclerosis and depression.

## Patentansprüche

1. Verbindung der Formel (I) 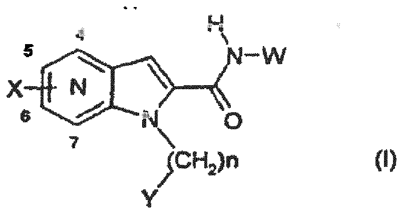 worin
n gleich 0, 1, 2 oder 3 ist;
der Pyrrolopyridinkern eine Pyrrolo[3,2-b]pyridingruppe, eine Pyrrolo[3,2-c]pyridingruppe, eine Pyrrolo[2,3-c]pyridingruppe oder eine Pyrrolo[2,3-b]pyridingruppe ist;
wobei der Pyrrolopyridinkern gegebenenfalls in Kohlenstoffposition 4, 5, 6 und/oder 7 durch einen oder mehrere Substituenten X substituiert ist, die gleich oder voneinander verschieden sind und unter einem Halogenatom oder einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₃-alkylen-O-, C₁-C₆-Fluoralkoxy-, Cyano-, C(O)NR₁R₂-, Nitro- , NR₁R₂- , C₁-C₆-Thioalkyl-, -S(O)-C₁-C₆-Alkyl-, -S(O)₂-C₁-C₆-Alkyl-, SO₂NR₁R₂-, NR₃COR₄-, NR₃SO₂R₅-, Aryl-, Aryl-C₁-C₅-alkylen-, Heteroaryl- oder Heteroaryl-C₁-C₅-alkylengruppe ausgewählt sind, wobei das Aryl oder
das Heteroaryl gegebenenfalls durch einen oder mehrere Substituenten substituiert ist, die unter einem Halogen oder einer C₁-C₆-Alkyl-, C₃-C₇-Cycloalkyl-, C₃-C₇-Cycloalkyl-C₁-C₃-alkylen-O-, C₁-C₆-Fluoralkyl-, C₁-C₆-Alkoxy-, C₃-C₇-Cycloalkoxy-, C₃-C₇-Cycloalkyl-C₁-C₃-alkylen-O-, C₁-C₆-Fluoralkoxy-, Nitro- oder Cyanogruppe ausgewählt sind;
Y für ein Heteroaryl steht, das gegebenenfalls durch eine oder mehrere Gruppen substituiert ist, die unter einem Halogenatom oder einer C₁-C₆-Alkyl-, C₃-C₇-Cycloalkyl-, C₃-C₇-Cycloalkyl-C₁-C₃-alkylen-, C₁-C₆-Fluoralkyl-, Hydroxy-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Cyano-, C(O)NR₁R₂-, Nitro-, NR₁R₂-, C₁-C₆-Thioalkyl-, C₁-C₆-Thiofluor-alkyl-, Thiol-, -S(O)-C₁-C₆-Alkyl-, -S (O) -C₃-C₇-Cycloalkyl-, -S(O)-C₁-C₆-Alkylen-C₃-C₇-cycloalkyl-, -S(O)₂-C₁-C₆-Alkyl-, -S(O)₂-C₃-C₇-Cycloalkyl-, -S(O)₂-C₁-C₆-Alkylen-C₃-C₇-cycloalkyl-, SO₂NR₁R₂-, NR₃COR₄-, NR₃SO₂R₅-, Aryl-, Aryl-C₁-C₅-alkylen-, Heteroaryl- oder Heteroaryl-C₁-C₅-alkylengruppe ausgewählt sind, wobei das Aryl oder das Heteroaryl gegebenenfalls durch einen oder mehrere Substituenten substituiert ist, die unter einem Halogen oder einer C₁-C₆-Alkyl-, C₃-C₇-Cycloalkyl-,
C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind;
R₁ und R₂ unabhängig voneinander für ein Wasserstoffatom oder eine C₁-C₆-Alkyl-, C₃-C₇-Cycloalkyl-, C₃-C₇-Cycloalkyl-C₁-C₃-alkylen-, Aryl-, Aryl-C₁-C₅-alkylen-, Heteroaryl- oder Heteroaryl-C₁-C₅-alkylengruppe stehen, wobei das Aryl oder das Heteroaryl gegebenenfalls durch einen oder mehrere Substituenten substituiert ist, die unter einem Halogen oder einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind;
oder R₁ und R₂ gemeinsam mit dem Stickstoffatom, an das sie gebunden sind, eine Azetidin-Pyrrolidin-, Piperidin-, Azepin-, Morpholin-, Thiomorpholin-, Piperazin- oder Homopiperazingruppe bilden, wobei diese Gruppe gegebenenfalls durch eine C₁-C₆-Alkyl-, C₃-C₇-Cycloalkyl-, C₃-C₇-Cycloalkyl-C₁-C₃-alkylen-, Aryl-, Aryl-C₁-C₅-alkylen-, Heteroaryl- oder Heteroaryl-C₁-C₅-alkylengruppe substituiert ist, wobei das Aryl oder das Heteroaryl gegebenenfalls durch einen oder mehrere Substituenten substituiert ist, die unter einem Halogen oder einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind; R₃ und R₄ unabhängig voneinander für ein Wasserstoffatom oder eine C₁-C₆-Alkyl-, C₃-C₇-Cycloalkyl-, C₃-C₇-Cycloalkyl-C₁-C₃-alkylen-, Aryl-, Aryl-C₁-C₅-alkylen-, Heteroaryl- oder Heteroaryl-C₁-C₅-alkylengruppe stehen, wobei das Aryl oder das Heteroaryl gegebenenfalls durch einen oder mehrere Substituenten substituiert ist, die unter einem Halogen oder einer C₁-C₆-Alkyl-, C₃-C-₇-Cycloalkyl-, C₃-C₇-Cycloalkyl-C₁-C₃-alkylen-, C₁-C₆-Fluoralkyl-C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind;
R₅ für eine C₁-C₆-Alkyl-, C₃-C₇-Cycloalkyl-, C₃-C₇-Cycloalkyl-C₁-C₃-alkylen-, Aryl-, Aryl-C₁-C₅-alkylen-, Heteroaryl- oder Heteroaryl-C₁-C₅-alkylengruppe steht, wobei das Aryl oder das Heteroaryl gegebenenfalls durch einen oder mehrere Substituenten substituiert ist, die unter einem Halogen oder einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind;
W für eine anellierte bicyclische Gruppe der Formel: 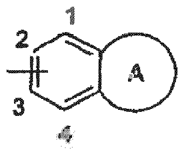 steht, die über die Positionen 1, 2, 3 oder 4 an das Stickstoffatom gebunden ist;
A für einen 5- bis 7-gliedrigen Heterocyclus mit einem bis drei unter O, S und N ausgewählten Heteroatomen steht;
wobei das Kohlenstoffatom bzw. die Kohlenstoffatome von A gegebenenfalls durch eine oder mehrere Gruppen substituiert ist bzw. sind, die unter einem Wasserstoffatom oder einer C₁-C₆-Alkyl-, C₃-C₇-Cycloalkyl-, C₃-C₇-Cycloalkyl-C₁-C₃-alkylen-, C₁-C₆-Fluoralkyl-, Aryl-, Aryl-C₁-C₅-alkylen-, Heteroaryl-, Heteroaryl-C₁-C₅-alkylen-, Oxo- oder Thiogruppe ausgewählt sind, wobei das Aryl oder das Heteroaryl gegebenenfalls durch einen oder mehrere Substituenten substituiert ist, die unter einem Halogen oder einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind; wobei das Stickstoffatom bzw. die Stickstoffatome von A dann, wenn der Stickstoff neben einem durch eine Oxogruppe substituierten Kohlenstoffatom steht, gegebenenfalls durch R₆ oder in den anderen Fällen durch R₇ substituiert ist bzw. sind;
R₆ für ein Wasserstoffatom oder eine C₁-C₆-Alkyl-, C₃-C₇-Cycloalkyl-, C₃-C₇-Cycloalkyl-C₁-C₃-alkylen-, C₁-C₆-Fluoralkyl-, Aryl-, Aryl-C₁-C₅-alkylen-, Heteroaryl- oder Heteroaryl-C₁-C₅-alkylengruppe steht, wobei das Aryl oder das Heteroaryl gegebenenfalls durch einen oder mehrere Substituenten substituiert ist, die unter einem Halogen oder einer C₁-C₆-Alkyl-, C₃-C₇-Cycloalkyl-, C₃-C₇-Cycloalkyl-C₁-C₃-alkylen-, C₁-C₆-Fluoralkyl-, C₁-C₆-Alkoxy-, C₃-C₇-Cycloalkoxy-, C₃-C₇-Cycloalkyl-C₁-C₆-alkylen-O-, C₁-C₆-Fluoralkoxy-, Nitro- oder Cyanogruppe ausgewählt sind;
R₇ für ein Wasserstoffatom oder eine C₁-C₆-Alkyl-, C₃-C₇-Cycloalkyl-, C₃-C₇-Cycloalkyl-C₁-C₃-alkylen-, C₁-C₆-Fluoralkyl-, Aryl-C₁-C₆-alkylen-, C₁-C₆-Alkyl-C(O)-, C₃-C₇-CyCloalkyl-C₁-C₃₋alkylen-C(O) -, C₁-C₆-Fluoralkyl-C(O) -, C₃-C₇-Cycloalkyl-C(O) -, Aryl-C(O)-, Aryl-C₁-C₆-alkylen-C (O) -, C₁-C₆-Alkyl-S(O)₂-, C₁-C₆-Fluoralkyl-S(O)₂-, C₃-C₇-Cycloalkyl-S(O)₂-, C₃-C₇-Cycloalkyl-C₁-C₃-alkylen-S(O)₂-, Aryl-S(O)₂-, Aryl-C₁-C₆-alkylen-S(O)₂- oder Arylgruppe steht;
wobei das Schwefelatom bzw. die Schwefelatome des Heterocyclus A in oxidierter Form vorliegen kann bzw. können;
wobei das Stickstoffatom bzw. die Stickstoffatome des Heterocyclus A in oxidierter Form vorliegen kann bzw. können;
das Stickstoffatom in Position 4, 5, 6 oder 7 des Pyrrolopyridins in oxidierter Form vorliegen kann; in Basen- oder Säureadditionssalzform sowie in Hydrat- oder Solvatform.

2. Verbindung der Formel (I) nach Anspruch 1, **dadurch gekennzeichnet, dass** n gleich 0 oder 1 ist; in Basen- oder Säureadditionssalzform sowie in Hydrat- oder Solvatform.

3. Verbindung der Formel (I) nach Anspruch 1 oder 2, **dadurch gekennzeichnet, dass** der Pyrrolopyridinkern eine Pyrrolo[3,2-*b*]pyridingruppe, eine Pyrrolo[3,2-*c*]pyridingruppe, eine Pyrrolo[2,3-*c*]-pyridingruppe oder eine Pyrrolo[2,3-*b*]pyridingruppe ist;
wobei der Pyrrolopyridinkern gegebenenfalls in Kohlenstoffposition 4, 5, 6 und/oder 7 durch einen oder mehrere Substituenten X substituiert ist, die gleich oder voneinander verschieden sind und unter einem Halogenatom oder einer C₁-C₆-Alkyl- oder C₁-C₆-Fluoralkylgruppe ausgewählt sind; in Basen- oder Säureadditionssalzform sowie in Hydrat- oder Solvatform.

4. Verbindung der Formel (I) nach Anspruch 1 oder 2, **dadurch gekennzeichnet, dass** der Pyrrolopyridinkern eine Pyrrolo[3,2-b]pyridingruppe, eine Pyrrolo[3,2-c]pyridingruppe, eine Pyrrolo[2,3-c]-pyridingruppe oder eine Pyrrolo[2,3-b]pyridingruppe ist;
wobei der Pyrrolopyridinkern gegebenenfalls in Kohlenstoffposition 5 durch einen Substituenten X gemäß der Definition in der allgemeinen Formel (I) gemäß Anspruch 1 substituiert ist; in Basen- oder Säureadditionssalzform sowie in Hydrat- oder Solvatform.

5. Verbindung der Formel (I) nach einem der Ansprüche 1 bis 3, **dadurch gekennzeichnet, dass** der Pyrrolopyridinkern eine Pyrrolo[2,3-b]pyridingruppe ist, die gegebenenfalls in Kohlenstoffposition 4, 5, 6 und/oder 7 durch einen oder mehrere Substituenten X substituiert ist, die gleich oder voneinander verschieden sind und unter einem Halogenatom oder einer C₁-C₆-Fluoralkylgruppe ausgewählt sind; in Basen- oder Säureadditionssalzform sowie in Hydrat- oder Solvatform.

6. Verbindung der Formel (I) nach Anspruch 5, **dadurch gekennzeichnet, dass** der Pyrrolopyridinkern eine Pyrrolo[2,3-b]pyridingruppe ist, die gegebenenfalls in Kohlenstoffposition 5 durch einen Substituenten X, der unter einem Halogenatom und einer C₁-C₆-Fluoralkylgruppe ausgewählt ist, substituiert ist; in Basen- oder Säureadditionssalzform sowie in Hydrat- oder Solvatform.

7. Verbindung der Formel (I) nach einem der Ansprüche 1 bis 6, **dadurch gekennzeichnet, dass** Y für eine Gruppe steht, die unter Imidazolyl-, Thiazolyl-, Oxazolyl-, Furanyl-, Thiophenyl-, Oxadiazolyl-, Tetrazolyl-, Pyridinyl-, Pyrazinyl-, Pyrimidinyl-, Pyridazinyl-, Indolyl-, Benzofuranyl-, Benzothiophenyl-, Benzoxazolyl-, Benzimidazolyl-, Indazolyl-, Benzothiazolyl-, Isobenzothiazolyl-, Benzotriazolyl-, Chinolinyl-, Isochinolinyl-, Chinoxalinyl-, Cinnolinyl-, Chinazolinyl-, Phthalazinyl- und Naphthyridinylgruppen ausgewählt ist; wobei diese Gruppe gegebenenfalls wie in der allgemeinen Formel (I) gemäß Anspruch 1 definiert substituiert ist; in Basen- oder Säureadditionssalzform sowie in Hydrat- oder Solvatform.

8. Verbindung der Formel (I) nach Anspruch 7, **dadurch gekennzeichnet, dass** Y für ein Pyridinyl steht; in Basen- oder Säureadditionssalzform sowie in Hydrat- oder Solvatform.

9. Verbindung der Formel (I) nach einem der Ansprüche 1 bis 8, **dadurch gekennzeichnet, dass** W unter Indolinyl-, Isoindolinyl-, Indolyl-, Isoindolyl-, Benzofuranyl-, Dihydrobenzofuranyl-, Benzothiophenyl-, Dihydrobenzothiophenyl-, Benzoxazolyl-, Dihydrobenzoxazolinyl-, Isobenzofuranyl-, Dihydroisobenzofuranyl-, Benzimidazolyl-, Dihydrobenzimidazolyl-, Indazolyl-, Benzothiazolyl-, Isobenzothiazolyl-, Dihydroisobenzothiazolyl-, Benzotriazolyl-, Chinolinyl-, Dihydrochinolinyl-, Tetrahydrochinolinyl-, Isochinolinyl-, Dihydroisochinolinyl-, Tetrahydroisochinolinyl-, Benzoxazinyl-, Dihydrobenzoxazinyl-,
Benzothiazinyl-, Dihydrobenzothiazinyl-, Cinnolinyl-, Chinazolinyl, Dihydrochinazolinyl-, Tetrahydrochinazolinyl-, Chinoxalinyl-, Dihydrochinoxalinyl-, Tetrahydrochinoxalinyl-, Phthalazinyl-, Dihydrophthalazinyl-, Tetrahydrophthalazinyl-, Tetrahydrobenz[*b*]azepinyl-, Tetrahydrobenz[*c*]azepinyl-, Tetrahydrobenz[d]azepinyl-, Tetrahydrobenzo[b]-[1,4]diazepinyl-, Tetrahydrobenzo[*e*][1,4]-diazepinyl-, Tetrahydrobenzo[*b*] [1,4]oxazepinyl- oder Tetrahydrobenzo[b] [1,4]thiazepinylgruppen ausgewählt ist; wobei diese Gruppen gegebenenfalls wie in der allgemeinen Formel (I) gemäß Anspruch 1 definiert substituiert sein können; in Basen- oder Säureadditionssalzform sowie in Hydrat- oder Solvatform.

10. Verbindung der Formel (I) nach Anspruch 9, **dadurch gekennzeichnet, dass** W für eine Benzimidazolyl- oder Benzothiazolylgruppe steht, wobei das Kohlenstoffatom bzw. die Kohlenstoffatome von W gegebenenfalls durch eine oder mehrere C₁-C₆-Alkylgruppen substituiert ist bzw. sind und das Stickstoffatom bzw. die Stickstoffatome von W gegebenenfalls durch eine C₁-C₆-Alkylgruppe substituiert ist bzw. sind; in Basen- oder Säureadditionssalzform sowie in Hydrat- oder Solvatform.

11. Verfahren zur Herstellung einer Verbindung der Formel (I) nach einem der Ansprüche 1 bis 10, **dadurch gekennzeichnet, dass** man eine Verbindung der allgemeinen Formel (IV) 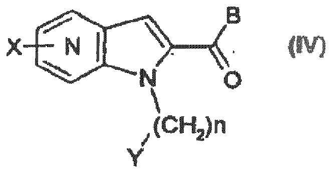 worin n, X und Y die in der allgemeinen Formel (I) gemäß Anspruch 1 angegebene Bedeutung besitzen und B für eine C₁-C₄-Alkoxygruppe steht,
am Rückflusspunkt eines Lösungsmittels mit einem Amid der Verbindung der allgemeinen Formel (V) 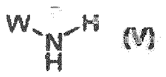 worin W die in der allgemeinen Formel (I) gemäß Anspruch 1 angegebene Bedeutung besitzt, umsetzt, wobei das Amid der Verbindung der allgemeinen Formel (V) durch vorherige Einwirkung von Trimethylaluminium auf die Verbindungen der allgemeinen Formel (V) hergestellt wird.

12. Verfahren zur Herstellung einer Verbindung der Formel (I) nach einem der Ansprüche 1 bis 10, **dadurch gekennzeichnet, dass** man eine Verbindung der allgemeinen Formel (IV) 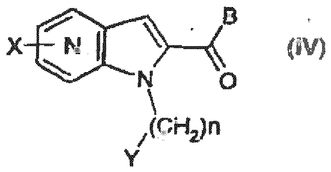 worin n, X und Y die in der allgemeinen Formel (I) gemäß Anspruch 1 angegebene Bedeutung besitzen und B für eine Hydroxylgruppe steht,
durch Einwirkung von Thionylchlorid am Rückflusspunkt eines Lösungsmittels in das Säurechlorid umwandelt
und dann die erhaltene Verbindung der allgemeinen Formel (IV), worin die in der allgemeinen Formel (I) gemäß Anspruch 1 angegebene Bedeutung besitzen und B für ein Chloratom steht, in Gegenwart einer Base mit der Verbindung der allgemeinen Formel (V) 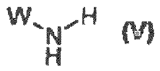 worin W die in der allgemeinen Formel (I) gemäß Anspruch 1 angegebene Bedeutung besitzt, umsetzt oder eine Kupplungsreaktion zwischen einer Verbindung der allgemeinen Formel (IV), worin n, X und Y die in der allgemeinen Formel (I) gemäß Anspruch 1 angegebene Bedeutung besitzen und B für eine Hydroxylgruppe steht,
und der Verbindung der allgemeinen Formel (V), worin W die in der allgemeinen Formel (I) gemäß Anspruch 1 angegebene Bedeutung besitzt, in Gegenwart eines Kupplungsmittels und einer Base in einem Lösungsmittel durchführt.

13. Arzneimittel, **dadurch gekennzeichnet, dass** es eine Verbindung der Formel (I) nach einem der Ansprüche 1 bis 10 oder ein pharmazeutisch annehmbares Salz oder auch ein Hydrat oder ein Solvat der Verbindung der Formel (I) enthält.

14. Pharmazeutische Zusammensetzung, **dadurch gekennzeichnet, dass** sie eine Verbindung der Formel (I) nach einem der Ansprüche 1 bis 10 oder ein pharmazeutisch annehmbares Salz, ein Hydrat oder ein Solvat dieser Verbindung sowie mindestens einen pharmazeutisch annehmbaren Hilfsstoff enthält.

15. Verwendung einer Verbindung der Formel (I) nach einem der Ansprüche 1 bis 10 zur Herstellung eines Arzneimittels zur Prävention oder Behandlung von Pathologien, an denen die Rezeptoren vom TRPV1-Typ beteiligt sind.

16. Verwendung einer Zusammensetzung der Formel (I) nach einem der Ansprüche 1 bis 10 zur Herstellung eines Arzneimittels zur Prävention oder Behandlung von Schmerzen, Entzündungen, Stoffwechselstörungen, urologischen Störungen, gynäkologischen Störungen, gastrointestinalen Störungen, respiratorischen Störungen, Psoriasis, Pruritis, Haut-, Augen- oder Schleimhautreizungen, Herpes, Zona, multipler Sklerose und Depression.
